(19) Europäisches Patentamt
European Patent Office
Office européen des brevets

(11) Veröffentlichungsnummer: 0 389 898 A2

# (12) EUROPÄISCHE PATENTANMELDUNG

(21) Anmeldenummer: 90105051.8

(22) Anmeldetag: 17.03.90

(51) Int. Cl.5: C07K 5/02, C07K 5/06, C07D 233/64, A61K 37/64

Die Bezeichnung der Erfindung wurde geändert (Richtlinien für die Prüfung im EPA, A-III, 7.3).

(30) Priorität: 28.03.89 CH 1118/89

(43) Veröffentlichungstag der Anmeldung: 03.10.90 Patentblatt 90/40

(84) Benannte Vertragsstaaten: AT BE CH DE DK ES FR GB GR IT LI LU NL SE

(71) Anmelder: F. HOFFMANN-LA ROCHE AG
Postfach 3255
CH-4002 Basel(CH)

(72) Erfinder: Branca, Quirico, Dr.
Lenzgasse 2
CH-4006 Basel(CH)

Erfinder: Märki, Hans Peter, Dr.
Seltisbergerstrasse 75
CH-4059 Basel(CH)
Erfinder: Neidhart, Werner, Dr.
Oltmannstrasse 14
D-7800 Freiburg im Breisgau(DE)
Erfinder: Ramuz, Henri, Prof. Dr.
Rheinparkstrasse 3
CH-4127 Birsfelden(CH)
Erfinder: Wostl, Wolfgang, Dr.
Im Strick 2
D-7889 Grenzach-Wyhlen(DE)

(74) Vertreter: Kellenberger, Marcus, Dr. et al
Grenzacherstrasse 124 Postfach 3255
CH-4002 Basel(CH)

## (54) Peptidartige Aminosäurederivate mit reninhemmender Wirkung.

(57) Die Verbindungen der Formel

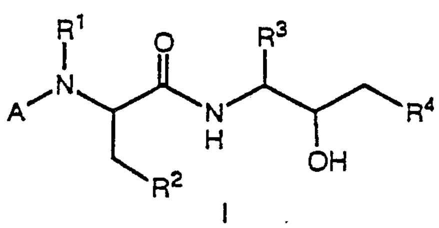

I

worin A, $R^1$, $R^2$, $R^3$ und $R^4$ die in Anspruch 1 angegebene Bedeutung besitzen, in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können nach verschiedenen, an sich bekannten Verfahren hergestellt werden.

EP 0 389 898 A2

## Peptidartige Aminosäurederivate

Die vorliegende Erfindung betrifft Aminosäurederivate. Im speziellen betrifft sie Aminosäurederivate der allgemeinen Formel

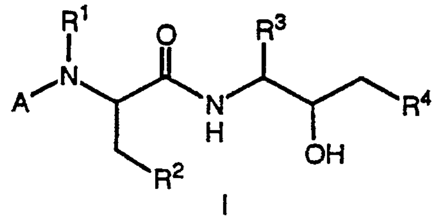

I

worin $R^1$ Wasserstoff oder Methyl, $R^2$ Aethyl, Propyl, Isopropyl, Imidazol-2-yl, Imidazol-4-yl. Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Nitro, Amino oder eine Gruppe der Formel -N($R^5$)($R^6$) und A eine der Gruppen

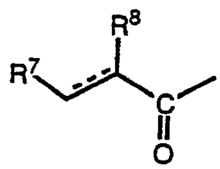

und -Y-Z (b)

(a)

bedeuten, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl, gegebenenfalls substituiertes Benzimidazolonyl oder den Rest einer gegebenenfalls acylierten Aminosäure oder eines gegebenenfalls acylierten Dipeptids oder $R^5$ und $R^6$ zusammen mit dem sie verbindenden Stickstoffatom einen 5- oder 6-gliedrigen Heterocyclus, ein 5- oder 6-gliedriges Lactam oder ein 5- oder 6-gliedriges Imid bedeuten, mit der Massgabe, dass A nicht die Gruppe (b) bedeuten kann, wenn $R^6$ Alkanoyl, Alkoxycarbonyl oder Arylalkoxycarbonyl bedeutet, die gestrichelte Linie eine zusätzliche Bindung bedeuten kann, $R^7$ Phenyl, substituiertes Phenyl, Benzyl oder Naphthyl und $R^8$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^8$ nicht Alkoxycarbonylamino oder Arylalkoxycarbonylamino bedeuten kann, wenn $R^7$ Phenyl, Benzyl oder $\alpha$-Naphthyl bedeutet, Y den N-terminal mit Z verbundenen, bivalenten Rest von gegebenenfalls N- und/oder $\alpha$-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, $\alpha$-Naphthylalanin oder Homophenylalanin und Z Wasserstoff oder Acyl bedeuten,
in Form von optisch reinen Diastereomeren, Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser

Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Die nachstehenden Definitionen der in der vorliegenden Beschreibung verwendeten allgemeinen Ausdrücke besitzen ihre Gültigkeit unabhängig davon, ob die fraglichen Ausdrücke allein oder in Kombination aufscheinen.

Der in der vorliegenden Beschreibung verwendete Ausdruck "Alkyl" bedeutet geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Isobutoxy, sec.-Butoxy, t-Butoxy und dergleichen. Der Ausdruck "Cycloalkyl" bedeutet gesättigte, cyclische Kohlenwasserstoffreste mit 3-8, vorzugsweise 3-6, Kohlenstoffatomen, wie Cyclopropyl, Cyclobutyl, Cyclopentyl, Cyclohexyl und dergleichen. Der Ausdruck "Heterocycloalkyl" betrifft in ähnlicher Weise gesättigte, 3-8-gliedrige, vorzugsweise 5- oder 6-gliedrige, cyclische Kohlenwasserstoffreste, in welchen eine oder zwei Methylengruppen durch ein oder zwei Sauerstoff- , Schwefel- oder gegebenenfalls durch Alkyl, Phenylalkyl, Alkanoyl oder Alkanoyloxy substituierte Stickstoffatome ersetzt sind, wie Piperidinyl, Pyrazinyl, N-Benzylpyrazinyl, Morpholinyl, N-Methylpiperidinyl, N-Benzylmorpholinyl und dergleichen. Der Ausdruck "Alkanoyl" bedeutet den Säurerest einer geradkettigen oder verzweigten Alkansäure mit 1-8, vorzugsweise 1-4, Kohlenstoffatomen, wie Formyl, Acetyl, Propionyl, Butyryl, Valeryl, Isovaleryl und dergleichen. Der Ausdruck "Aryl" bezeichnet einen gegebenenfalls durch Alkyl, Alkoxy, Alkanoyloxy, Amino, Alkylamino, Dialkylamino, Alkylcarbonylamino, Hydroxy, Halogen, Trifluormethyl oder Nitro ein- oder mehrfach substituierten mono- oder bicyclischen aromatischen Kohlenwasserstoffrest mit 6-14 Kohlenstoffatomen, wie Phenyl, α- oder β-Naphthyl, Indenyl, Anthryl oder Phenanthryl und dergleichen. Der Ausdruck "Arylalkyl" bezeichnet geradkettige oder verzweigte Alkylgruppen, worin ein oder mehrere Wasserstoffatome durch Arylgruppen ersetzt sind, wie Benzyl, Diphenylmethyl, Trityl, α- oder β-Naphthylmethyl, 2-Phenyläthyl, 3-Phenyl-2-propyl, 4-Phenyl-3-butyl, 2-(α- oder β-Naphthyl)äthyl, 3-α-Naphthyl-2-propyl, 4-α-Naphthyl-3-butyl und dergleichen, wobei der aromatische Rest jeweils wie oben ange geben ein- oder mehrfach substituiert sein kann. Der Ausdruck "substituiertes Phenyl" bezeichnet gegebenenfalls durch Alkyl, Alkoxy, Alkoxyalkoxy, Alkanoyl, Alkanoyloxy, Hydroxy, Halogen oder Trifluormethyl ein- oder mehrfach substituiertes Phenyl, wie 4-Hydroxyphenyl, 4-Methoxyphenyl, 4-Methylphenyl, 4-Chlorphenyl, 4-Aethoxyäthoxyphenyl und dergleichen. Beispiele für gegebenenfalls substituiertes Benzimidazolonyl sind Benzimidazolonyl, 3-Methylbenzimidazolonyl, 3-Isopropylbenzimidazolonyl, 3-Butylbenzimidazolonyl, 3-Morpholinoäthylbenzimidazolonyl, 3-Benzylbenzimidazonyl und dergleichen. Der Ausdruck "5- oder 6-gliedriger Heterocyclus" betrifft gesättigte 5- oder 6-gliedrige Heterocyclen mit mindestens einem Stickstoffatom und gegebenenfalls einem zusätzlichen Sauerstoff-, Stickstoff- oder Schwefelatom als Ringglieder, wie Piperidinyl, Pyrazinyl, Morpholinyl, Thiomorpholinyl, Pyrrolidinyl, Thiazolidinyl, Imidazolidinyl, Oxazolidinyl und dergleichen. Der Ausdruck "5- oder 6-gliedriges Lactam" betrifft cyclische Amide geradkettiger oder verzweigter Alkansäuren. Der Ausdruck "5- oder 6-gliedriges Imid" betrifft in ähnlicher Weise cyclische Diamide von zweibasischen Säuren, wie Bernsteinsäure, Glutarsäure, Phthalsäure und dergleichen.

Der Ausdruck "substituiertes Amino" bedeutet eine durch Alkyl, Arylalkyl, Alkanoyl, Alkoxycarbonyl oder Arylalkoxycarbonyl mono- oder di-substituierte oder eine gegebenenfalls durch ein Sauerstoff-, Schwefel- oder gegebenenfalls Alkyl-, Phenylalkyl-, Alkanoyl- oder Alkanoyloxy-substituiertes Stickstoffatom unterbrochene $C_3$-$C_6$-Alkylen disubstituierte Aminogruppe. Der Ausdruck "Acyl" betrifft die Acylgruppe einer Carbonsäure, eines Halbesters der Kohlensäure, einer gegebenenfalls N-substituierten Carbamin- oder Thiocarbaminsäure, eines gegebenenfalls N-substituierten Oxalamids, einer Sulfonsäure oder einer gegebenenfalls N-substituierten Amidosulfonsäure, insbesondere solche mit den Teilformeln $R^b$-CO-, $R^a$-O--CO-, $(R^b)(R^b)$N-CO-, $(R^b$ $(R^b)$N-CS-, $(R^b)(R^b)$N--CO-CO-, $R^b$-$SO_2$-, oder $(R^b)(R^b)$N-$SO_2$-, worin $R^a$ einen unsubstituierten oder substituierten, gesättigten oder ungesättigten, gegebenenfalls mit Amino, Monoalkylamino, Dialkylamino, Alkanoylamino oder Alkanoyloxyamino funktionalisierten aliphatischen, cycloaliphatischen, cycloaliphatisch-aliphatischen Kohlenwasserstoffrest mit bis zu 18, vorzugsweise 10, Kohlenstoffatomen, einen unsubstituierten oder substituierten aromatischen, heteroaromatischen, aromatisch-aliphatischen oder heteroaromatisch-aliphatischen Kohlenwasserstoffrest mit bis zu 18, vorzugsweise 10, Kohlenstoffatomen oder einen unsubstituierten oder substituierten, gesättigten 5- oder 6-gliedrigen heterocyclischen Rest bedeutet und $R^b$ Wasserstoff bedeutet oder die Bedeutung von $R^a$ besitzt. Der Ausdruck "Acyl" betrifft auch den einwertigen, über die Carboxylgruppe gebundenen Rest einer gegebenenfalls acylierten Aminosäure oder eines gegebenenfalls acylierten Dipeptids.

Ein unsubstituierter oder substituierter, gesättigter oder ungesättigter, aliphatischer, cycloaliphatischer oder cycloaliphatisch-aliphatischer Kohlenwasserstoffrest $R^a$ oder $R^b$ ist beispielsweise unsubstituiertes oder substituiertes Alkyl, Alkenyl, Alkinyl, Mono-, Bi- oder Tricycloalkyl, Monocycloalkenyl, Bicycloalkenyl, Cycloalkylalkyl, Cycloalkylalkenyl oder Cycloalkenylalkyl. "Substituiertes Alkyl" bedeutet einen Alkylrest, in welchem ein oder mehrere Wasserstoffatome durch Hydroxy, Alkoxy, Aryloxy, Alkanoyloxy, Halogen, Hydroxysulfonyloxy, Carboxy, Alkoxycarbonyl, Carbamoyl, Alkylcarbamoyl, Dialkylcarbamoyl, Cyan, Phosphono, verestertes Phosphono, Amino oder Oxo substituiert sein können, wobei die Substituenten nur dann in 1-Stellung des Alkylrestes stehen, wenn dieser in der Teilformel $R^b$--CO- an die Carbonylgruppe gebunden ist.

Beispiele von substituiertem Alkyl sind 2-Hydroxyäthyl, Methoxymethyl, 2-Methoxyäthyl, Phenoxymethyl, α- oder β-Naphthoxymethyl, Acetoxymethyl, 2-Acetoxyäthyl, Chlormethyl, Brommethyl, 2-Chlor- oder 2-Bromäthyl, Hydroxysulfonyloxymethyl, 2-Hydroxysulfonyloxyäthyl, Carboxymethyl, 2-Carboxyäthyl, Methoxycarbonylmethyl, 2-Methoxycarbonyläthyl, Aethoxycarbonylmethyl, 2-Aethoxycarbonyläthyl, Carbamoylmethyl, 2-Carbamoyläthyl, Methylcarbamoylmethyl, Dimethylcarbamoylmethyl, Cyanomethyl, 2-Cyanoäthyl, 2-Oxopropyl, 2-Oxobutyl, Hydroxycarboxymethyl, 1-Hydroxy-2-carboxyäthyl, Hydroxyäthoxycarbonyläthyl, Hydroxymethoxycarbonyläthyl, Acetoxymethoxycarbonylmethyl, 1,2-Dihydroxy-2-carboxyäthyl, 1,2-Dihydroxy-2-äthoxycarbonyläthyl, 1,2-Dihydroxy-2-methoxycarbonyläthyl, 1,2-Diacetoxy-2-äthoxycarbonyläthyl, 1,2-Diacetoxy-2-methoxycarbonyläthyl, 1-α-Naphthoxy-3-carboxypropyl, 1-α-Naphthoxy-2-äthoxycarbonyläthyl, 1-α-Naphthoxy-3-t-butoxycarbonylpropyl, 1-α-Naphthoxy-2-benzyloxycarbonyläthyl, 1-α-Naphthoxy-3-carbamoylpropyl, α-Naphthoxycyanomethyl, 1-α-Naphthoxy-3-cyanopropyl, 1-α-Naphthoxy-4-dimethylaminobutyl oder 1-α-Naphthoxy-3-oxobutyl.

Der Ausdruck "Alkenyl" betrifft geradkettige oder verzweigte, ungesättigte Kohlenwasserstoffreste mit 2-8, vorzugsweise 2-4, Kohlenstoffatomen, wobei die Doppelbindung nur dann in 1-Stellung des Alkenylrestes steht, wenn dieser in der Teilformel $R^b$-CO- an die Carbonylgruppe gebunden ist. Beispiele solcher Alkenylreste sind Vinyl, Allyl, 2-Butenyl oder 3-Butenyl. Die Alkenylreste können durch die gleichen Substituenten substituiert sein wie die Alkylreste.

Der Ausdruck "Alkinyl" betrifft Kohlenwasserstoffreste mit 2-8, vorzugsweise 2-4, Kohlenstoffatomen, welche eine Dreifachbindung enthalten, wie Aethinyl, 1-Propinyl oder 2-Propinyl. Der Ausdruck "Bicycloalkyl" betrifft bicyclische gesättigte Kohlenwasserstoffreste mit 5-10, vorzugsweise 6-9, Kohlenstoffatomen, wie Bicyclo[3.1.O]hex-1-yl, Bicyclo [3.1.O]hex-2-yl, Bicyclo[3.1.O]hex-3-yl, Bicyclo[4.1.O]hept-1-yl, Bicyclo[4.1.O]hept-4-yl, Bicyclo[2.2.1]hept-2-yl, Bicyclo[3.2.1]oct-2-yl, Bicyclo[3.3.O]oct-3-yl, Bicyclo[3.3.1]-non-9-yl, α- oder β-Decahydronaphthyl und dergleichen.

Der Ausdruck "Tricycloalkyl" betrifft einen tricyclischen gesättigten Kohlenwasserstoffrest mit 8-10 Kohlenstoffatomen, wie 1-Adamantyl.

Der Ausdruck "Cycloalkenyl" betrifft einen ungesättigten cyclischen Kohlenwasserstoffrest mit 3-8, vorzugsweise 3-6, Kohlenstoffatomen, wie 1-Cyclohexenyl, 1,4-Cyclohexadienyl und dergleichen.

Der Ausdruck "Bicycloalkenyl" betrifft einen bicyclischen ungesättigten Kohlenwasserstoffrest mit 5-10, vorzugsweise 7-10, Kohlenstoffatomen, wie 5-Norbornen-2-yl, Bicyclo[2.2.2]octen-2-yl, Hexahydro-4,7-methanoind-1-en-6-yl und dergleichen.

Beispiele für Cycloalkylalkyl sind Cyclopropylmethyl, Cyclobutylmethyl, Cyclopentylmethyl, Cyclohexylmethyl und dergleichen. Als Beispiele für Cycloalkylalkenyl können Cyclohexylvinyl und Cyclohexylallyl und dergleichen genannt werden. Beispiele für Cycloalkenylalkyl sind 1-Cyclohexenylmethyl, 1,4-Cyclohexadienylmethyl und dergleichen.

Die genannten cycloaliphatischen und cycloaliphatisch-aliphatischen Reste können durch die gleichen Substituenten wie Alkyl substituiert sein .

Ein gegebenenfalls substituierter aromatischer oder aromatisch-aliphatischer Kohlenwasserstoffrest ist beispielsweise unsubstituiertes oder substituiertes Aryl, Arylalkyl oder Arylalkenyl. Beispiele für Arylalkenyl sind Styryl, 3-Phenylallyl, 2-(α-Naphthyl)vinyl, 2-(β-Naph thyl)vinyl und dergleichen.

In einem heteroaromatischen oder heteroaromatisch-aliphatischen Kohlenwasserstoffrest ist der Heterocyclus mono-, bi- oder tricyclisch und enthält ein bis zwei Stickstoffatome und/oder ein Sauerstoff- oder Schwefelatom und ist mit einem seiner Ring-Kohlenstoffatome mit der Gruppe -CO-, -O-CO-, >N-CO-, >N-CS-, >N-CO-CO-, $-SO_2$ oder >N-$SO_2$-verknüpft. Beispiele solcher heteroaromatischer Kohlenwasserstoffreste sind Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanneliertes, cyclopenta-, cyclohexa- oder cyclohepta-anneliertes Derivat dieser Reste. Der heteroaromatische Rest kann an einem Stickstoffatom durch Alkyl, Phenyl oder Phenylalkyl, z.B. Benzyl und/oder an einem oder mehreren Kohlenstoffatomen durch Alkyl, Phenyl, Phenylalkyl, Halogen, Hydroxy, Alkoxy, Phenylalkoxy oder Oxo substituiert und teilweise gesättigt sein. Beispiele solcher heteroaromatischer Reste sind 2- oder 3-Pyrrolyl, Phenylpyrrolyl,

z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl und dergleichen.

Beispiele heteroaromatisch-aliphatischer Kohlenwasserstoffreste sind 2- oder 3-Pyrrolylmethyl, 2-, 3- oder 4-Pyridylmethyl, 2-(2-, 3- oder 4-Pyridyl)äthyl, 4-Imidazolylmethyl, 2-(4-Imidazolyl)äthyl, 2-Indolylmethyl, 3-Indolylmethyl, 2-(3-Indolyl)äthyl, 2-Chinolylmethyl und derglei chen.

Ein gesättigter 5- oder 6-gliedriger heterocyclischer Rest hat mindestens ein Kohlenstoffatom, 1-3 Stickstoffatome und gegebenenfalls ein Sauerstoff- oder Schwefelatom als Ringglieder und ist mit einem seiner Ringkohlenstoffatome mit der Gruppe -CO- bzw. -O-CO-, >N-CO-, >N-CS-, >N-CO-CO-, $-SO_2-$ oder $>N-SO_2-$ verknüpft. Der Heterocyclus kann an einem seiner Kohlenstof fatome oder an einem Ringstickstoffatom durch Alkyl, z.B. Methyl oder Aethyl, Phenyl oder Phenylalkyl, z.B. Benzyl, oder an einem seiner Kohlenstoffatome durch Hydroxy oder Oxo substituiert und/oder an zwei benachbarten Kohlenstoffatomen benzanneliert sein. Beispiele solcher Reste sind Pyrrolidin-3-yl, 4-Hydroxypyrrolidin-2-yl, 5-Oxopyrrolidin-2-yl, Piperidin-2-yl, Piperidin-3-yl, 1-Methylpiperidin-2-yl, 1-Methylpiperidin-3-yl, 1-Methylpiperidin-4-yl, Morpholin-2-yl, Morpholin-3-yl, Thiomorpholin-2-yl, Thiomorpholin-3-yl, 1,4-Dimethylpiperazin-2-yl, 2-Indolinyl, 3-Indolinyl, 1,2,3,4-Tetrahydrochinol-2-, -3- oder -4-yl, 1,2,3,4-Tetrahydroisochinol-1-, -3-oder -4-yl, 1-Oxo-1,2,3,4-tetrahydroisochinol-3-yl und dergleichen.

Als Rest einer über die Carboxylgruppe gebundenen Aminosäure kommen natürliche α-Aminosäuren mit der L-Konfiguration, Homologe solcher Aminosäuren, z.B. worin die Aminosäurenseitenkette um eine oder zwei Methylengruppen verlängert oder verkürzt ist und/oder eine Methylgruppe durch Wasserstoff ersetzt ist, substituierte aromatische α-Aminosäuren, z.B. substituiertes Phenylalanin oder Phenylglycin, worin der Substituent Alkyl, z.B. Methyl, Halogen, z.B. Fluor, Chlor, Brom oder Jod, Hydroxy, Alkoxy, z.B. Methoxy, Alkanoyloxy, z.B. Acetoxy, Amino, Alkylamino, z.B. Methylamino, Dialkylamino, z.B. Dimethylamino, Alkanoylamino, z.B. Acetylamino oder Pivaloylamino, Alkoxycarbonylamino, z.B. t-Butoxycarbonylamino, Arylmethoxycarbonylamino, z.B. Benzyloxycarbonylamino und/oder Nitro sein kann und ein- oder mehrfach vorkommt, benzanneliertes Phenylalanin oder Phenylglycin, wie α-Naphthylalanin, oder hydriertes Phenylalanin oder Phenylglycin, wie Cyclohexylalanin oder Cyclohexylglycin, eine 5- oder 6-gliedrige cyclische benzannelierte α-Aminosäure, z.B. Indolin-2-carbonsäure oder 1,2,3,4-Tetrahydroisochinolin-3-carbonsäure, eine natürliche oder homologe α-Aminosäure, in der eine Carboxygruppe in der Seitenkette in veresterter oder amidierter Form vorliegt, z.B. als Alkylestergruppe, wie Methoxycarbonyl oder t-Butoxycarbonyl, oder als Carbamoyl-, Alkylcarbamoyl, wie Methylcarbamoyl, oder als Dialkylcarbamoylgruppe, wie Dimethylcarbamoyl, in der eine Aminogruppe der Seitenkette in acylierter Form vorliegt, z.B. als Alkanyolamino, wie Acetylamino oder Pivaloylamino, als Alkoxycarbonylamino-, wie t-Butoxycarbonylamino, oder als Arylmethoxycarbonylaminogruppe, wie Benzyloxycarbonylamino, oder in der eine Hydroxygruppe der Seitenkette in verätherter oder veresterter Form vorliegt, z.B. als Alkoxygruppe, wie Methoxy, als Arylalkoxygruppe, wie Benzyloxy, oder als nieder Alkanoyloxygruppe, wie Acetoxy, oder Epimere solcher Aminosäuren, d.h. mit der unnatürlichen D-Konfiguration. Beispiele solcher Aminosäuren sind Glycin, Alanin, Valin, Norvalin, Leucin, Isoleucin, Norleucin, Serin, Homoserin, Threonin, Methionin, Cystein, Prolin, Trans-3- und Trans-4-hydroxyprolin, Phenylalanin, Tyrosin, 4-Nitrophenylalanin, 4-Aminophenylalanin, 4-Chlorphenylalanin, β-Phenylserin, Phenylglycin, α-Naphthylalanin, Cyclohexylalanin, Cyclohexylglycin, Tryptophan, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutaminsäuremono-t-butylester, Glutamin, N-Dimethylglutamin, Histidin, Arginin, Lysin, N-t-Butoxycarbonyllysin, δ-Hydroxylysin, Ornithin, N-Pivaloylornithin, α,γ-Diaminobuttersäure oder α,β-Diaminopropionsäure und dergleichen. Der über die Carboxylgruppe gebundene Rest der Aminosäure kann N-terminal zur Erhöhung der Stabilität der Verbindung der Formel I gegen enzymatischen Abbau durch Alkyl, z.B. Methyl oder Aethyl, substituiert sein.

Der über die Carboxylgruppe gebundene Rest eines Dipeptids besteht aus zwei der oben erwähnten Aminosäuren.

Der Ausdruck "acylierte Aminosäure" bzw. "acyliertes Dipeptid" betrifft eine der oben erwähnten Aminosäuren bzw. ein Dipeptid aus zwei der oben erwähnten Aminosäuren, welche bzw. welches N-terminal durch den Acylrest einer Carbonsäure, eines Halbesters der Kohlensäure, einer gegebenenfalls N-substituierten Carbamin- oder Thiocarbaminsäure, eines gegebenenfalls N-substituierten Oxalamids, einer Sulfonsäure oder einer gegebenenfalls N-substituierten Amidosulfonsäure substituiert ist.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugt sind solche Verbindungen der Formel I, worin $R^1$ Wasserstoff bedeutet. $R^2$ bedeutet vorzugsweise Imidazol-2-yl, Imidazol-4-yl oder Thiazol-4-yl, besonders bevorzugt Imidazol-4-yl. Weiter sind solche Verbindungen der Formel I bevorzugt, worin $R^3$ Cyclohexylmethyl bedeutet. $R^4$ bedeutet vorzugsweise $-N(R^5)(R^6)$. $R^5$ bedeutet vorzugsweise Alkyl, besonders bevorzugt Methyl, und $R^6$ vorzugsweise den Rest einer gegebenenfalls acylierten Aminosäure oder eines gegebenenfalls acylierten Dipeptids, besonders bevorzugt den acylierten Rest von Histidin oder Phenylalanin oder den acylierten Rest des Dipeptids aus Histidin und Phenylalanin, oder $R^5$ und $R^6$ zuammen mit dem sie verbindenden Stickstoffatom bedeuten ein 5- oder 6-gliedriges Lactam. Ebenfalls bevorzugt sind die Verbindungen der Formel I, worin A die Gruppe (a) bedeutet. $R^7$ bedeutet vorzugsweise Phenyl oder substituiertes Phenyl, besonders bevorzugt Phenyl. Die bevorzugte Bedeutung von $R^8$ ist Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, vorzugsweise Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl. Falls A die Gruppe (b) bedeutet, so sind diejenigen Verbindungen der Formel I bevorzugt, worin Y den N-terminal mit Z verbundenen, bivalenten Rest von Phenylalanin bedeutet. Z bedeutet vorzugsweise die Gruppe $R^a$-O-CO-, worin $R^a$ einen gegebenenfalls substituierten, gesättigten aliphatischen Kohlenwasserstoffrest mit bis zu 10 Kohlenstoffatomen oder einen gegebenenfalls substituierten heteraromatischen Kohlenwasserstoffrest mit bis zu 18 Kohlenstoffatomen bedeutet, ganz besonders bevorzugt die Gruppe $R^a$-O-CO-, worin $R^a$ einen gesättigten, aliphatischen Kohlenwasserstoffrest mit bis zu 6 Kohlenstoffatomen oder einen heteroaromatischen Rest mit bis 10 Kohlenstoffatomen bedeutet.

Aus dem obigen folgt, dass solche Verbindungen der Formel I ganz besonders bevorzugt sind, worin $R^1$ Wasserstoff, $R^2$ Imidazol-4-yl, $R^3$ Cyclohexylmethyl, $R^4$ $-N(R^5)(R^6)$, $R^5$ Methyl, $R^6$ den acylierten Rest von Histidin oder Phenylalanin oder den acylierten Rest des Dipeptids aus Histidin und Phenylalanin, $R^7$ Phenyl und $R^8$ $C_1$-$C_4$-Alkylcarbonylmethyl oder $C_1$-$C_4$-Alkylsulfonylmethyl bedeuten.

Ganz speziell bevorzugte Verbindungen der Formel I sind:
(S)-N-[(1S,2S)-1-(Cyclohexylmethyl)-2-hydroxy-3-(2-oxopiperidino)propyl]-α-[(R)-α-(3, 3-dimethyl-2-oxobutyl)hydrocinnamamido]imidazol-4-propionamid,
(S)-N-[(1S,2S)-1-(Cyclohexylmethyl)-3-[(S)-α-[(R)-α-(3,3-dimethyl-2-oxobutyl)hydrocinnamamidoj-N-isopropylimidazol-4-propionamido]-2-hydroxypropyl]-α-[(R)-α-(3,3-dimethyl-2-oxobutyl)hydrocinnamamido]-imidazol-4-propionamid,
(S)-N-[(1S,2S)-1-(Cyclohexylmethyl)-2-hydroxy-3-[[(S)-1-[(S)-α-(3,3-dimethyl-2-oxobutyl)hydrocinnamamido]-2imidazol-4-yläthyl]methylamino]propyl]-α-[(R)-α-(3,3-dimethyl-2-oxobutyl)hydrocinnamamido]imidazol-4-propionamid,
(S)-N-[(1S,2S)-1-(Cyclohexylmethyl)-2-hydroxy-3-[(S)-3-(imidazol-4-yl)-2-hydrocinnamamido-N-methylpropionamido]propyl]-α-[(R)-α-(3,3-dimethyl-2-oxobutyl)hydrocinnamamido]imidazol-4-propionamid,
t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]carbamoyl]-2-imidazol-4-yläthyl]carbamoyl]-phenäthyl]carbamat,
t-Butyl [(S)-α-[[(2S,3S)-3-[(S)-2-[(S)-α-(1-t-butoxyformamido)hydrocinnamamido]-3-imidazol-4-ylpropionamido]-4-cyclohexyl-2-hydroxybutyl]methylcarbamoyl]phenäthyl]carbamat und
(S)-N-[(1S,2S)-1-(Cyclohexylmethyl)-2-hydroxy-3-phthalimidopropyl]-α-[(R)-α-(3,3-dimethyl-2-oxobutyl)-hydrocinn amamido]imidazol-4-propionamid.

Die Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man

a) eine Verbindung der allgemeinen Formel

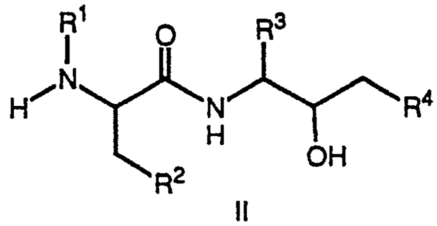

II

worin $R^1$, $R^2$, $R^3$ und $R^4$ die oben angegebene Bedeutung besitzen, mit einem die Gruppe

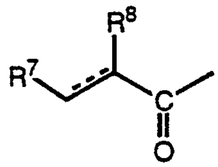

(a)

oder -Y-Z (b)

worin $R^7$, $R^8$, Y, Z und die gestrichelte Linie die oben angegebene Bedeutung besitzen, abgebenden Acylierungsmittel umsetzt, oder

b) eine Verbindung der allgemeinen Formel

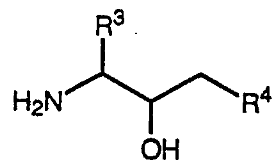

III

worin $R^3$ und $R^4$ die oben angegebene Bedeutung besitzen, mit einer Verbindung der allgemeinen Formel

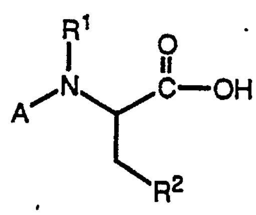

IV

worin $R^1$, $R^2$ und A die oben angegebene Bedeutung besitzen, oder einem aktivierten Derivat davon umsetzt, oder

c) eine Verbindung der Formel I, worin Z Wasserstoff bedeutet und die übrigen Symbole die oben angegebene Bedeutung besitzen, mit einer gegebenenfalls acylierten Aminosäure oder einem gegebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeutet, aus einer entsprechenden Verbindung der Formel I, worin A eine N-geschützte Aminogruppe enthält, und/oder aus einer der Formel I entsprechenden Verbindung, worin $R^4$ jedoch N-gechütztes Amino und/oder $R^2$ N-geschütztes Imidazol-2-yl, Imidazol-4-yl oder Pyrazol-3-yl bedeutet, die N-Schutzgruppe(n) abspaltet, oder

e) zur Herstellung einer Verbindung der Formel I, worin $R^4$ ein 5- oder 6-gliedriges Imid bedeutet,

eine entsprechende Verbindung der Formel I, worin $R^4$ Amino bedeutet, mit einem Anhydrid einer zweibasischen Säure umsetzt, oder

f) zur Herstellung einer Verbindung der Formel I, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl oder den Rest einer gegebenenfalls acylierten Aminosäure oder eines gegebenenfalls acylierten Dipeptids bedeuten, eine der Formel I entsprechende Verbindung, worin $R^6$ jedoch Wasserstoff und $R^5$ Alkyl, Alkoxyalkyl oder gegebenenfalls substituiertes Phenyl, Phenylalkyl oder Phenylsulfonylalkyl bedeuten, mit einem den Rest $R^6$ abgebenden Acylierungsmittel umsetzt, und

g) erwünschtenfalls ein Gemisch diastereomerer Racemate in die diastereomeren Racemate oder optisch reinen Diastereomeren auftrennt, und/oder

h) erwünschtenfalls ein Diastereomerengemisch in die optisch reinen Diastereomeren auftrennt, und/oder

i) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0° C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Falls es sich beim Acylierungsmittel um ein Peptid handelt, erfolgt die Reaktion unter in der Peptidchemie üblichen Reaktionsbedingungen, d.h. vorzugsweise in Gegen wart eines Kondensationsmittels wie HBTU (O-Benzotriazolyl-N,N,N′,N′-tetramethyluronium-hexafluorophosphat), BOP (Benzotriazol-1-yloxy-bis-(dimethylamino)phosphonium-hexafluorophosphat), BOPC (Bis(2-oxo-2-oxozolidinyl)phosphinchlorid), HOBT (N-Hydroxybenzotriazol), DBU (1,8-Diazabicyclo[5.4.O]undec-7-en), DCC (Dicyclohexylcarbodiimid), EDC (N-Aethyl-N′(3-dimethylaminopropyl)carbodiimid-hydrochlorid), Hünigbase (Aethyldiisopropylamin), und dergleichen. Die Reaktion wird zweckmässigerweise in einem unter den Reaktionsbedingungen inerten organischen Lösungsmittel oder Lösungsmittelgemisch bei einer Temperatur zwischen etwa 0° und 50° C, vorzugsweise bei etwa Raumtemperatur durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel III mit einer Verbindung der Formel IV erfolgt ebenfalls nach an sich in der Peptid-Chemie bekannten Methoden, d.h. unter den gleichen Bedingungen wie oben für die Umsetzung einer Verbindung der Formel II mit einem Peptid angegeben wurde. Beispiele geeigneter aktivierter Derivate einer Verbindung der Formel IV sind Säurehalogenide, Säureanhydride, gemischte Anhydride, Ester, gemischte Ester, und dergleichen.

Die Umsetzung einer Verbindung der Formel I, worin Z Wasserstoff bedeutet, mit einer gegebenenfalls acylierten Aminosäure oder einem gegebenenfalls acylierten Dipeptid gemäss Verfahrensvariante c) erfolgt ebenfalls nach an sich in der Peptid-Chemie bekannten Methoden, d.h. unter den oben für die Umsetzung einer Verbindung der Formel II mit einem Peptid angegebenen Bedingungen.

Die Abspaltung der N-Schutzgruppe(n) gemäss Verfahrensvariante d) erfolgt ebenfalls nach an sich bekannten Methoden in Abhängigkeit von der Art der abzuspaltenden N-Schutzgruppe. Die Abspaltung erfolgt jedoch zweckmässig durch saure oder basische Hydrolyse. Für die saure Hydrolyse verwendet man vorteilhafter Weise eine Lösung einer Mineralsäure, wie Chlorwasserstoffsäure, Bromwasserstoffsäure, Trifluoressigsäure, Schwefelsäure, Phosphorsäure und dergleichen, in einem inerten Lösungsmittel oder Lösungsmittelgemisch. Geeignete Lösungsmittel sind Alkohole, wie Methanol oder Aethanol, Aether, wie Tetrahydrofuran oder Dioxan, chlorierte Kohlenwasserstoffe, wie Methylenchlorid, und dergleichen. Für die basische Hydrolyse können Alkalimetallhydroxyde und -carbonate, wie Kalium- oder Natriumhydroxyd oder Kalium- oder Natriumcarbonat, organische Amine, wie Piperidin, und dergleichen verwendet werden. Inerte organische Lösungsmittel, wie sie oben für die saure Hydrolyse genannt sind, können als Lösungsvermittler zugegeben werden. Die Reaktionstemperatur kann für die saure und basische Hydrolyse in einem Bereich von etwa 0° C bis Rückflusstemperatur variiert werden, wobei man vorzugsweise zwischen etwa 0° C und der Raumtemperatur arbeitet. Der t-Butoxycarbonylrest wird zweckmässig mit Trifluoressigsäure oder Ameisensäure in Gegenwart oder Abwesenheit eines inerten Lösungsmittels abgespalten. Die Fmoc-Schutzgruppe wird zweckmässig mit Piperidin bei etwa Raumtemperatur abgespalten. Die Benzyloxycarbonylgruppe kann in bekannter Weise durch saure Hydrolyse wie weiter oben beschrieben oder hydrogenolytisch abgespalten werden.

Die Umsetzung einer Verbindung der Formel I, worin $R^4$ Amino bedeutet, mit einem Anhydrid einer

zweibasischen Säure erfolgt ebenfalls nach an sich bekannten Methoden in einem unter den Reaktionsbedingungen inerten organischen Lösungsmittel bei einer Temperatur zwischen etwa Raumtemperatur und der Rückflusstemperatur. Geeignete Lösungsmittel sind aromatische Kohlenwasserstoffe, wie Toluol oder Xylol, Acetonitril, Dimethylformamid und dergleichen.

Die Acylierung einer der Formel I entsprechenden Verbindung, worin $R^6$ jedoch Wasserstoff und $R^5$ Alkyl, Alkoxy alkyl oder gegebenenfalls substituiertes Phenyl, Phenylalkyl oder Phenylsulfonylalkyl bedeuten, mit einem den Rest $R^6$ abgebenden Acylierungsmittel erfolgt ebenfalls nach an sich bekannten Methoden. Geeignete Acylierungsmittel sind Säurehalogenide, Säureanhydride, gemischte Anhydride, Säureazide, Ester, gemischte Ester und dergleichen. Die Umsetzung erfolgt in einem unter den Reaktionsbedingungen inerten organischen Lösungsmittel oder Lösungsmittelgemisch bei einer Temperatur zwischen etwa Raumtemperatur und der Rückflusstemperatur des Reaktionsgemisches, vorzugsweise bei etwa Raumtemperatur. Die Reaktion kann in Gegenwart oder Abwesenheit eines Säurebindemittels, wie Natrium- oder Kaliumcarbonat, Pyridin, Triäthylamin und dergleichen, durchgeführt werden.

Die Ausgangsstoffe der Formel II sind neu und ebenfalls Gegenstand der vorliegenden Erfindung. Diese Verbindungen können hergestellt werden, indem man eine Verbindung der Formel III mit gegebenenfalls N-methyliertem Histidin, Leucin, Norleucin, Norvalin, Thiazolylalanin, Thienylalanin, Asparaginsäureäthylester, Glutaminsäure-t-butylester, Glutaminsäurebenzylester oder t-Butoxyserin umsetzt. Diese Umsetzung erfolgt ebenfalls nach in der Peptid-Chemie bekannten Methoden, d.h. unter den Reaktionsbedingungen, wie sie weiter oben für die Umsetzung einer Verbindung der Formel II mit einem Dipeptid beschrieben sind.

Die Ausgangsstoffe der Formel III sind ebenfalls neu und Gegenstand der vorliegenden Erfindung. Sie können beispielsweise hergestellt werden, indem man in einer Verbindung der allgemeinen Formel

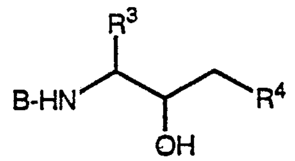

V

worin B eine Aminoschutzgruppe bedeutet, vorzugsweise t-Butoxycarbonyl oder Benzyloxycarbonyl, und $R^3$ und $R^4$ die oben angegebene Bedeutung besitzen,
die Aminoschutzgruppe abspaltet.

Die Abspaltung der N-Schutzgruppe erfolgt ebenfalls nach an sich bekannten Methoden, beispielsweise in einem unter den Reaktionsbedingungen inerten organischen Lösungsmittel oder Lösungsmittelgemisch bei einer Temperatur zwischen etwa O° C und der Raumtemperatur mit einer Säure, wie Chlorwasserstoffsäure, Trifluoressigsäure, und dergleichen. Geeignete Lösungsmittel sind Aether, wie Tetrahydrofuran oder Dioxan, Alkohole, wie Methanol, oder chlorierte Kohlenwasserstoffe, wie Methylenchlorid, und dergleichen.

Die Ausgangsstoffe der Formel IV sind bekannt oder können in Analogie zur Herstellung der bekannten Verbindungen erhalten werden.

Die Verbindungen der Formel V sind ebenfalls neu und Gegenstand vorliegender Erfindung. Diejenigen, worin $R^4$ Amino oder die Gruppe $-N(R^5)(R^6)$ bedeutet, können beispielsweise durch Umsetzen einer Aminoverbindung der allgemeinen Formel

$HN(R^5)(R)^6$ VI

worin $R^5$ und $R^6$ die oben angegebene Bedeutung besitzen,
mit einem Epoxyd der allgemeinen Formel

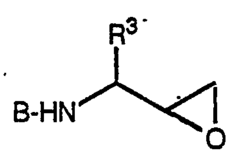

VII

worin B und $R^3$ die oben angegebene Bedeutung besitzen, hergestellt werden.

Die Umsetzung einer Aminoverbindung der Formel VI mit einem Epoxyd der Formel VII erfolgt ebenfalls nach an sich bekannten Methoden, beispielsweise in einem unter den Reaktionsbedingungen inerten organischen Lösungsmittel oder Lösungsmittelgemisch bei einer Temperatur zwischen etwa Raumtemperatur und der Rückflusstemperatur. Geeignete Lösungsmittel sind Alkohole, wie Methanol oder Aethanol, Aether, wie Diäthyläther, Tetrahydrofuran oder Dioxan, und dergleichen oder Gemische davon.

Die Verbindungen der Formel V, worin $R^4$ Nitro bedeutet, können ebenfalls nach an sich bekannten Methoden hergestellt werden, beispielsweise durch Umsetzen eines Aldehyds der allgemeinen Formel

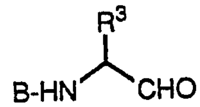

VIII

worin B und $R^3$ die oben angegebene Bedeutung besitzen, mit Nitromethan in Gegenwart einer starken Base, wie Natriumhydrid, Natriumamid, Kalium-t-butylat und dergleichen, in einem unter den Reaktionsbedingungen inerten organischen Lösungsmittel, wie einem Aether, z.B. Diäthyläther, Tetrahydrofuran oder Dioxan, einem Alkohol, z.B. t-Butylalkohol, und dergleichen, bei einer Temperatur zwischen etwa Raumtemperatur und der Rückflusstemperatur, vorzugsweise zwischen etwa 30° und 50° C.

Die Verbindungen der Formeln VI, VII und VIII sind bekannt oder können in Analogie zur Herstellung der bekannten Verbindungen hergestellt werden.

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

In vitro-Test mit reinem Human-Renin

Der Test wird in Eppendorf Röhrchen durchgeführt. Die Inkubationsmischung besteht aus (1) 100 µl Human-Renin in Puffer A (0,1M Natriumphosphatlösung, pH 7,4, enthaltend 0,1% Rinderserumalbumin, 0,1 % Natriumazid und 1 mM Aethy lendiamintetraessigsäure), genügend für eine Renin-Aktivität von 2-3 ng Angiotensin I/ ml/Std.; (2) 145 µl Puffer A; (3) 30 µl von 10 µM humanem Tetradekapeptid-Reninsubstrat (hTD) in 10 mM Salzsäure; (4) 15 µl Dimethylsulfoxid mit bzw. ohne Hemmer und (5) 10 µl einer 0,03 molaren Lösung von Hydroxychinolinsulfat in Wasser.

Die Proben werden drei Stunden bei 37° C bzw. 4° C in Triplikaten inkubiert. 2 x 100 µl Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100 %; Angiotensin II 0,0013 %; hTD (Angiotensin I-Val-Ile-His-Ser-OH) 0,09 % Die Produktion von Angiotensin I wird durch die Differenz zwischen dem Versuch bei 37° C und demjenigen bei 4° C bestimmt.

Folgende Kontrollen werden mitgeführt:

(a) Inkubation von hTD-Proben ohne Renin und ohne Hemmer bei 37° C und 4° C. Die Differenz zwischen diesen beiden Werten ergibt den Grundwert der Angiotensin I-Produktion.

(b) Inkubation von hTD-Proben mit Renin, jedoch ohne Hemmer bei 37° C und 4° C. Die Differenz

zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$- Werte werden aus einer linearen Regressionskurve aus einem logit-log plot ermittelt.

Die in diesem Test erhaltenen Resultate sind in der folgenden Tabelle zusammengefasst:

## Tabelle

| Verbindung | $IC_{50}$-Werte in µMol/lt. |
|---|---|
| A | 0,041 |
| B | 0,041 |
| C | 0,028 |
| D | 0,019 |
| E | 0,030 |
| F | 0,015 |
| G | 0,020 |

A= (S)-N-[(1S,2S)-1-(Cyclohexylmethyl)-2-hydroxy-3-(2-oxopiperidino)propyl]-α-[(R)-α-(3,3-dimethyl-2-oxobutyl)-hydrocinnamamido]imidazol-4-propionamid,

B= (S)-N-[(1S,2S)-1-(Cyclohexylmethyl)-3-[(S)-α-[(R)-α-(3,3-dimethyl-2-oxobutyl)hydrocinnamamido]-N-isopropylimidazol-4-propionamido]-2-hydroxypropyl]-α-[(R)-α-(3,3-dimethyl-2-oxobutyl)hydrocinnamamido]imidazol-4-propionamid,

C= (S)-N-[(1S,2S)-1-(Cyclohexylmethyl)-2-hydroxy-3-[[(S)-1-[(S)-α-(3,3-dimethyl-2-oxobutyl)hydrocinnamamido]-2-imidazol-4-yläthyl]methylamino]propyl]-α-[(R)-α-(3,3-dimethyl-2-oxobutyl)hydrocinnamamido]imidazol-4-propionamid,

D= (S)-N-[(1S,2S)-1-(Cyclohexylmethyl)-2-hydroxy-3-[(S)-3-(imidazol-4-yl)-2-hydrocinnamamido-N-methylpropionamido]-propyl]-α-[(R)-α-(3,3-dimethyl-2-oxobutyl)hydrocinnamamido]imidazol-4-propionamid,

E= t-Butyl [(S)-α-[[(S)-1-[[(1S,2S)-3-[[(S)-1-[(S)-α-(1-t-butoxyformamido)hydrocinnamamido]-2-imidazol-4-yl-äthyl]methylcarbamoyl]-1-(cyclohexylmethyl)-2-hydroxypropyl]carbamoyl]-2-imidazol-4-yläthyl]carbamoyl]phen-äthyl]carbamat,

F= t-Butyl [(S)-α-[[(2S,3S)-3-[(S)-2-[(S)-α-(1-t-butoxyformamido)hydrocinnamamido]-3-imidazol-4-ylpropionamido]-4-cyclohexyl-2-hydroxybutyl]methylcarbamoyl]phenäthyl]-carbamat und

G= (S)-N-[(1S,2S)-1-(Cyclohexylmethyl)-2-hydroxy-3-phthalimidopropyl]-α-[(R)-α-(3,3-dimethyl-2-oxobutyl)hydrocinn-amamido]imidazol-4-propionamid.

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen verwendet:

H-His-OH = L-Histidin

H-Phe-OH = L-Phenylalanin
H-Phe-His-OH = N-[(S)-2-Amino-3-phenylpropyl]-L-histidin
Boc = t-Butoxycarbonyl
Fmoc = 9-Fluorenylmethoxycarbonyl

## Beispiel 1

63 mg (0,248 mMol) α-[(S)-1-Amino-2-cyclohexyläthyl]-1-piperidinäthanol (αR:αS = 5:1) in 10 ml Acetonitril werden mit 0,085 ml Hünigbase, 110 mg BOP und 100 mg Boc-Phe-His-OH versetzt. Die erhaltene Lösung wird anschliessend 12 Stunden bei Raumtemperatur gerührt. Nach üblicher Aufarbeitung wird das Rohprodukt zur Reinigung mit einem 10:1-Gemisch von Methylenchlorid und Methanol als Eluierungsmittel an Kieselgel chromatographiert, wobei man 50 mg (33%) t-Butyl [(S)-α-[[(S)-1-[[[1S,(2R:2S = 5:1)]-1-(cyclohexylmethyl)-2-hydroxy-3-piperidinopropyl]carbamoyl]-2-imidazol-4-yläthyl]carbamoyl]-phenäthyl]carbamat als Harz erhält, MS: 639 $(M + H)^+$.

In analoger Weise wurde durch Umsetzen von 183 mg (0,715 mMol) (R:S = 5:1)-α-[(S)-1-Amino-2-cyclohexyläthyl]morpholinäthanol mit 288 mg (0,715 mMol) Boc-Phe-His-OH 130 mg (28%) t-Butyl [(S)-α-[[-(S)-1-[[(1S,2R:2S = 5:1)-1-(cyclohexylmethyl)-2-hydroxy-3-morpholinopropyl] carbamoyl]-2-imidazol-4-yläthyl]carbamoyl]phenäthyl]carbamat als Schaum erhalten, MS: 641 $(M + H)^+$.

Das als Ausgangsstoff eingesetzte α-[(S)-1-Amino-2-cyclohexyläthyl]-1-piperidinäthanol (αR:αS = 5:1) wurde wie folgt hergestellt:

600 mg (2,22 mMol) t-Butyl [(1S,2R:S = 9:1)-1-(cyclohexylmethyl)-2,3-epoxypropyl]carbamat, welches nach der von S.W. Rosenberg et al. in J. Med. Chem. 30, 1224 (1987) beschriebenen Methode aus 2-t-Butoxycarbonylamino-3(S)-cyclohexylpropylaldehyd [welcher seinerseits nach der von J. Boger et al. in J. Med. Chem., 28, 1779 (1985) beschriebenen Methode hergestellt worden war] hergestellt worden war, in 10 ml Aethanol werden mit 0,658 ml (6,7 mMol) Piperidin versetzt, und das erhaltene Gemisch anschliessend 12 Stunden zum Rückfluss erhitzt. Nach dem Abkühlen gibt man Aether und Wasser zu, trennt die beiden Phasen, trocknet die organische Phase über Natriumsulfat und chromatographiert den verbleibenden Rückstand nach Entfernen des Lösungsmittels unter vermindertem Druck mit einem 10:1-Gemisch von Chloroform und Methanol als Eluierungsmittel an Kieselgel. Auf diese Weise werden 600 mg (76%) t-Butyl [(1S)-1-(cyclohexylmethyl)-2-hydroxy-3-piperidinopropyl]carbamat (2R:2S = 5:1) als weisser Festkörper erhalten, MS: 355 $(M + H)^+$.

600 mg (1,69 mMol) t-Butyl [(1S)-1-(cyclohexylmethyl)-2-hydroxy-3-piperidinopropyl]carbamat werden in 6 ml 5,2N Salzsäure in Dioxan gelöst und anschliessend 2 Stunden bei Raumtemperatur gerührt. Danach wird die Reaktionslösung unter vermindertem Druck eingedampft, mit 25%iger Ammoniaklösung alkalisch gestellt und mit Aether extrahiert. Trocken und Eindampfen der Aetherextrakte liefern 400 mg (93%) α-[(S)-1-Amino-2-cyclohexyläthyl]-1-piperidinäthanol (αR:αS = 5:1) als Harz, MS: 236 $(M - H_2O)^+$.

In analoger Weise wie oben beschrieben, wurden durch Umsetzen von 600 mg (2,22 mMol) t-Butyl [-(1S,2R:S = 9:1)-1-(cyclohexylmethyl)-2,3-epoxypropyl]carbamat mit 0,4 ml (4,4 mMol) Morpholin 700 mg (89%) t-Butyl [(1S,2R:2S = 5:1)-1-(cyclohexylmethyl-2-hydroxy-3-morpholinopropyl]carbamat als Oel erhalten, MS: 338 $(M - H_2O)^+$, die durch Rühren in 5,2N Salzsäure in Dioxan in 465 mg (90%) (R:S = 5:1)-α-[(S)-1-Amino-2-cyclohexyläthyl]morpholinäthanol in Form eines Oels übergeführt wurden, MS: 156 $(M - Morpholinomethyl)^+$.

## Beispiel 2

58 mg (0,2 mMol) α-[(S)-1-Amino-2-cyclohexyläthyl]-N-äthyl-N-phenylaminoäthanol, welches durch Abspalten der Boc-Schutzgruppe mit 5N Salzsäure in Dioxan aus t-Butyl [(1S,2R:S = 5:1)-1-(cyclohexylmethyl)-2-hydroxy-3-(äthylphe nylamino)propyl]carbamat [hergestellt aus t-Butyl [(1S, 2R:S = 9:1)-1-(cyclohexylmethyl)-2,3-epoxypropyl]carbamat und N-Aethylamidin in analoger Weise wie in Beispiel 1 beschrieben] erhalten wurde, werden in 5 ml Acetonitril gelöst und mit 42 ml Hünigbase, 91 mg BOP und 100 mg 1-(t-Butoxycarbonyl)-N-[(R)-α-(3,3-dimethyl-2-oxobutyl)hydrocinnamoyl]-L-histidin versetzt. Anschliessend wird das erhaltene Reaktionsgemisch 12 Stunden bei Raumtemperatur gerührt und dann unter vermindertem Druck eingedampft. Der Rückstand wird zwischen gesättigter Natriumbicarbonatlösung und Essigester verteilt, und die organische Phase abgetrennt, getrocknet und eingedampft. Chromatographie

des Rückstands an Kieselgel unter Verwendung eines 20:1-Gemisches von Methylenchorid und Methanol als Eluierungsmittel liefert 120 mg eines Produkts, welches zur Abspaltung der Boc-Schutzgruppe am Imidazolring in Methanol gelöst und nach Zugabe einer Spatelspitze Kaliumcarbonat 2 Stunden bei Raumtemperatur gerührt wird. Nach üblicher Aufarbeitung und Chromatographie des Rückstands an Kieselgel erhält man 76 mg (S)-N-[(1S,2R:2S=5:1)-1-(Cyclohexylmethyl)-3-(äthylphenylamino)-2-hydroxypropyl]-α-[(R)-α-(3,3-dimethyl-2-oxobutyl)hydrocinnamamido]imidazol-4-propionamid als Harz, MS: 658 (M + H)$^+$.

Das als Ausgangstoff eingesetzte 1-(t-Butoxycarbonyl)-N-[(R)-α-(3,3-dimethyl-2-oxobutyl)-hydrocinnamoyl]-L-histidin wurde wie folgt hergestellt:

Eine Suspension von 3,0 g (12 mMol) (R)-α-(Pivaloylmethyl)hydrozimtsäure (vgl. EPA 0.184.550) und 2,66 g (11 mMol) L-Histidinmethylester-dihydrochlorid in 340 ml Dimethylformamid wird bei Raumtemperatur unter einer Stickstoffatmosphäre mit 3,45 g (34 mMol) Triäthylamin und 4,58 g (12 mMol) HBTU versetzt. Das Reaktionsgemisch wird 5 Stunden bei Raumtemperatur gerührt und anschliessend im Hochvakuum eingedampft. Der Rückstand wird in 500 ml Essigester gelöst und nacheinander mit 100 ml Wasser, dreimal je 100 ml gesättigter Natriumbicarbonatlösung und 100 ml gesättigter Natriumchloridlösung gewaschen. Die organische Phase wird über Natriumsulfat getrocknet, unter vermin dertem Druck eingedampft, und das erhaltene gelbliche Rohprodukt an Kieselgel mit einem 95:5-Gemisch von Methylenchlorid und Methanol, welches 0,1% Ammoniak enthält, chromatographiert. Man erhält auf diese Weise 3,6 g N-[(R)-α-(3,3-Dimethyl-2-oxobutyl)hydrocinnamoyl]-L-histidinmethylester als farblosen Schaum, MS: 399 (M)$^+$.

Eine Lösung von 3,56 g (8,9 mMol) N-[(R)-α-(3,3-Dimethyl-2-oxobutyl)hydrocinnamoyl]-L-histidinmethylester und 9,36 ml 1N Natronlauge in 50 ml Methanol wird 15 Stunden bei Raumtemperatur gerührt und danach in der Kälte unter vermindertem Druck eingedampft. Man löst den Rückstand in 70 ml Dioxan und 30 ml Wasser, tropft bei Raumtemperatur eine Lösung von 2,95 (13,5 mMol) Di-t-butyldicarbonat zu und rührt danach 15 Stunden bei Raumtemperatur. Zur Aufarbeitung wird die Reaktionslösung unter vermindertem Druck auf ca. 1/3 ihres Volumens eingeengt und dann mit 200 ml Essigester verdünnt. Nach Zugabe von 50 ml Eiswasser wird das Reaktionsgemisch auf pH 2,5 gestellt, und die wässrige Phase mit festem Natriumchlorid gesättigt. Die wässrige Phase wird noch zweimal mit Essigester extrahiert, und die vereinigten Essigesterphasen über Natriumsulfat getrocknet und eingedampft. Das erhaltene Rohpodukt wird an Kieselgel mit einem 95:5-Gemisch von Methylenchlorid und Methanol, welches 0,1% Essigsäure enthält, chromatographiert, wobei man 3,5 g 1-(t-Butoxycarbonyl)-N-[(R)-α-(3,3-dimethyl-2-oxobutyl)-hydrocinnamoyl]-L-histidin als farbloses Pulver erhält, MS: 486 (M+H)$^+$.

## Beispiel 3

3,45 g (5 mMol) Benzyl [(2S,3S)-4-cyclohexyl-3-[(S)-α-[(R)-α-(3,3-dimethyl-2-oxobutyl)-hydrocinnamamido]imidazol-4-propionamido]-2-hydroxybutyl]carbamat in 50 ml Methanol werden mit 0,57 g Pyridinhydrochlorid versetzt und nach Zugabe von 0,5 g Palladium auf Kohle (5%) 4 Stunden hydriert. Danach wird der Katalysator abfiltriert, und das Filtrat unter vermindertem Druck eingedampft, wobei man 3,25 g (S)-N-[(1S,2S)-3-Amino-1-(cyclohexylmethyl)-2-hydroxypropyl]-α-[(R)-α-(3,3-dimethyl-2-oxobutyl)-hydrocinnamamido]imidazol-4-propionamid-hydrochlorid als amorphen Festkörper erhält, MS: 554 (M + H)$^+$.

Das als Ausgangsstoff eingesetzte Benzyl [(2S,3S)-4-cyclohexyl-3-[(S)-α-[(R)-α-(3,3-dimethyl-2-oxobutyl)hydrocinnamamido]imidazol-4-propionamido]-2-hydroxybutyl]carbamat wurde wie folgt hergestellt:

11,9 g (29,7 mMol) t-Butyl [(S)-3-amino-(S)-2-(t-butyldimethylsiloxy)-1-(cyclohexylmethyl)propyl]-carbamat (vgl. US Patentschrift 4.599.198) werden in 150 ml Dimethylformamid gelöst, mit 6,6 ml Hünigbase versetzt und auf 5° abgekühlt. Unter kräftigem Rühren werden bei dieser Temperatur innerhalb von 10 Minuten 4,65 ml Chlorameisensäurebenzylester zugetropft, und das Reaktionsgemisch anschliessend 1 Stunde bei Raumtemperatur gerührt. Danach wird das Reaktionsgemisch im Hockvakuum eingeengt, auf Wasser gegossen und mit Methylenchlorid extrahiert. Die Methylenchloridextrakte werden über Kaliumcarbonat getrocknet und eingedampft, und der Rückstand mit einem Gemisch von Methylenchlorid und Hexan als Eluierungsmittel an 150 g Kieselgel chromatographiert, wobei 11,05 g 3-Benzyl 1-t-butyl [-(1S,-2S)-2-(t-butyldimethylsiloxy)-1-(cyclohexylmethyl)propyl]-dicarbamat als Oel erhalten werden.

8,2 g (19,5 mMol) 3-Benzyl 1-t-butyl [(1S,2S)-2-(t-butyldimethylsiloxy)-1-(cyclohexylmethyl)propyl]-dicarbamat werden 100 ml Methylenchlorid gelöst, unter Eiskühlung mit 25 ml 90%iger Trifluoressigsäure versetzt und 4,5 Stunden bei Raumtemperatur gerührt. Das Reationsgemisch wird danach auf Eiswasser

gegossen, mit Natriumcarbonatlösung alkalisch gestellt und mit Methylenchlorid extrahiert. Die Methylenchloridextrakte werden über Kaliumcarbonat getrocknet und unter vermindertem Druck eingedampft. Chromatographie des erhaltenen Rückstands an 100 g Kieselgel mit einem Gemisch von Methylenchlorid und Isopropanol als Eluierungsmittel liefert 4,95 g Benzyl [(2S,3S)-3-amino-2-hydroxybutyl]carbamat als amorphen Festkörper, MS: 321 $(M + H)^+$.

Umsetzen von Benzyl [(2S,3S)-3-amino-2-hydroxybutyl]carbamat mit 1-(t-Butoxycarbonyl)-N-[(R)-α-(3,3-dimethyl-2-oxobutyl)hydrocinnamoyl]-L-histidin und Abspalten der Boc-Schutzgruppe am Imidazolring mit Kaliumcarbonat in Methanol, in analoger Weise wie in Beispiel 2 beschrieben, liefert Benzyl [(2S,3S)-4-cyclohexyl-3-[(S)-α-[(R)-α-(3,3-dimethyl-2-oxobutyl) hydrocinnamamido] imidazol-4-propionamido]-2-hydroxybutyl]carbamat als amorphen Festkörper, MS: 688 $(M + H)^+$.

## Beispiel 4

408 mg (0,565 mMol) (S)-N-[(1S,2S)-1-(Cyclohexylmethyl)-2-hydroxy-3-(2-oxo-1-pyrrolidinyl)propyl] -α-[-(R)-α-(3,3-dimethyl-2-oxobutyl)hydrocinnamamido]-3-t-butoxycarbonylimidazol-4-propionamid werden in 5,5 ml Methanol gelöst, mit 23 mg Kaliumcarbonat versetzt und 5 Stunden bei Raumtemperatur gerührt. Danach wird das Reaktionsgemisch auf Eiswasser gegossen und mit Methylenchlorid extrahiert. Die Methylenchloridextrakte werden über Kaliumcarbonat getrocknet und eingedampft, und der Rückstand an 35 g Kieselgel mit einem Gemisch von Methylenchlorid und Isopropanol als Eluierungsmittel chromatographiert, wobei man 280 mg (80%) (S)-N-[(1S,2S)-1-(Cyclohexylmethyl)-2-hydroxy-3-(2-oxo-1-pyrrolidinyl)-propyl]-α-[(R)-α-(3,3-dimethyl-2-oxobutyl)hydrocinnamamido]imidazol-4-propionamid als amorphen Festkörper erhält, MS: 622 $(M + H)^+$.

Das als Ausgangsstoff eingesetzte (S)-N-[(1S,2S)-1-(Cyclohexylmethyl)-2-hydroxy-3-(2-oxo-1-pyrrolidinyl)propyl]-α-[(R)-α-(3,3-dimethyl-2-oxobutyl)hydrocinnamamido]-3-t-butoxycarbonylimidazol-4-propionamid wurde wie folgt hergestellt:

1,6 g (4,0 mMol) t-Butyl [(S)-3-amino-(S)-2-(t-butyldimethylsiloxy)-1-(cyclohexylmethyl)propyl]carbamat werden in 20 ml Dimethylformamid gelöst und bei Raumtemperatur mit 1,12 ml Triäthylamin und 0,68 ml 4-Brombuttersäureäthylester versetzt. Das Reakionsgemisch wird danach 3 Stunden bei 95° gerührt, abgekühlt, anschliessend auf Eiswasser gegossen und mit Methylenchlorid extrahiert. Die Methylenchloridextrakte werden über Kaliumcarbonat getrocknet und unter vermindertem Druck eingedampft, und der Rückstand an 20 g Kieselgel mit einem Gemisch von Methylenchlorid und Isopropanol als Eluierungsmittel chromatographiert. Auf diese Weise erhält man 1,543 g t-Butyl [(1S,2S)-2-(t-butyldimethylsiloxy)-1-(cyclohexymethyl)-3-[[3-(äthoxycarbonyl)propyl]amino]propyl]carbamat als Oel, welches ohne weitere Reinigung in die nächste Stufe eingesetzt wird.

1,24 g (2,41 mMol) t-Butyl [(1S,2S)-2-(t-butyldimethylsiloxy)-1-(cyclohexymethyl)-3-[[3-(äthoxycarbonyl)-propyl]amino]propyl]carbamat werden in 15 ml Aethanol gelöst und mit 5,0 ml 1N Natronlauge versetzt. Das Reaktionsgemisch wird danach 2 Stunden bei Raumtemperatur gerührt, anschliessend auf Wasser gegossen, mit Essigsäure auf pH 4,5 angesäuert und mit Methylenchlorid extrahiert. Die Methylenchloridextrakte werden über Magnesiumsulfat getrocknet und unter vermindertem Druck eingedampft. Chromatographie des Rückstands an 15 g Kieselgel unter Verwendung eines Gemisches von Methylenchlorid und Methanol als Eluierungsmittel liefert 687 mg 4-[[(2S,3S)-3-(1-t-Butoxyformamido)-2-(t-butoxydimethylsiloxy)-3-(cyclohexylmethyl)propyl]amino]buttersäure in Form eines zähen Oels, welches direkt in die nächste Stufe eingesetzt wird.

0,6 g (1,23 mMol) 4-[[(2S,3S)-3-(1-t-Butoxyformamido)-2-(t-butoxydimethylsiloxy)-3-(cyclohexylmethyl)-propyl]amino]buttersäure wird in 2 ml Methylenchlorid gelöst und bei -20° zu einer Lösung von 285 mg EDC und 200 mg HOBT in 13 ml Methylenchlorid getropft. Das Reaktionsgemisch wird anschliessend innerhalb von 15 Minuten auf Raumtemperatur aufgewärmt, 17 Stunden bei dieser Temperatur gerührt und anschliessend auf Wasser gegossen und mit Methylenchlorid extrahiert. Die Methylenchloridextrakte werden über Magnesiumsulfat getrocknet, unter vermindertem Druck eingedampft, und der Rückstand an 10 g Kieselgel unter Verwendung eines Gemisches von Methylenchlorid und Isopropanol als Eluierungsmittel chromatographiert, wobei man 480 mg t-Butyl [(1S,2S)-2-(t-butyldimethylsiloxy)-1-(cyclohexylmethyl)-3-(2-oxo-1-pyrrolidinyl)propyl]carbamat als Oel erhält, welches direkt in die nächste Stufe eingesetzt wird.

0,62 g (1,28 mMol) t-Butyl [(1S,2S)-2-(t-butyldimethylsiloxy)-1-(cyclohexylmethyl)-3-(2-oxo-1-pyrrolidinyl)propyl]carbamat wird in einem Reaktionsgefäss aus Polypropylen in 9 ml Acetonitril gelöst und bei Raumtemperatur mit 1,2 ml 40%iger wässriger Fluorwasserstoffsäure versetzt. Das Reaktionsgemisch wird anschliessend 3 Stunden bei Raumtemperatur gerührt, danach auf 2N Natriumbicarbonatlösung gegossen

und mit Methylenchlorid extrahiert. Die Methylenchloridextrakte werden über Magnesiumsulfat getrocknet und unter vermindertem Druck eingedampft, wobei man 434 mg t-Butyl [(1S,2S)-1-(cyclohexylmethyl)-2-hydroxy-3-(2-oxo-1-pyrrolidinyl)propyl]carbamat als Oel erhält, welches direkt in die nächste Stufe eingesetzt wird.

0,41 g (1,16 mMol) t-Butyl [(1S,2S)-1-(cyclohexylmethyl)-2-hydroxy-3-(2-oxo-1-pyrrolidinyl)propyl]-carbamat wird in 5 ml Methylenchlorid gelöst, bei 0° mit 1,5 ml 90%iger Trifluoressigsäure versetzt und anschliessend 5 Stunden bei Raumtemperatur gerührt. Danach wird das Reaktionsgemisch langsam auf 2N Natriumcarbonatlösung getropft, anschliessend mit 3N Natronlauge versetzt und schliesslich mit Methylenchlorid extrahiert. Die Methylenchloridextrakte werden über Kaliumcarbonat getrocknet und unter vermindertem Druck eingedampft, wobei man 249 mg 1-[(2S,3S)-3-Amino-4-cyclohexyl-2-hydroxybutyl]-2-pyrrolidinon als Oel erhält, MS: 255 $(M + H)^+$.

220 mg (0,865 mMol) 1-[(2S,3S)-3-Amino-4-cyclohexyl-2-hydroxybutyl]-2-pyrrolidinon und 300 mg (0,618 mMol) 1-(t-Butoxycarbonyl)-N-[(R)-α-(3,3-dimethyl-2-oxobutyl)hydrocinnamoyl]-L-histidin werden in 5 ml Methylenchlorid gelöst, auf -20° gekühlt und mit 0,16 ml N,N-Diisopropyläthylamin und 0,12 ml Diäthylcyanophosphonat versetzt. Hierauf wird das Reaktionsgemisch 19 Stunden bei Raumtemperatur gerührt und anschliessend auf ein Gemisch von Eis und verdünnter Natriumbicarbonatlösung gegossen und mit Methylenchlorid extrahiert. Die Methylenchloridextrakte werden über Magnesiumsulfat getrocknet und eingedampft, und der Rückstand an 5 g Kieselgel mit einem Gemisch von Methylenchlorid und Isopropanol als Eluierungsmittel chromatographiert, wobei man 430 g (96%) (S)-N-[(1S,2S)-1-(Cyclohexylmethyl)-2-hydroxy-3-(2-oxo-1-pyrrolidinyl)propyl]-α-[(R)-α-(3,3-dimethyl-2-oxobutyl) hydrocinnamamido]-3-t-butoxycarbonylimidazol-4-propionamid als gelben Schaum erhält, welcher direkt in die nächste Stufe eingesetzt wird.

## Beispiel 5

In analoger Weise wie in Beispiel 4 beschrieben, wurde (S)-N-[(1S,2S)-1-(Cyclohexylmethyl)-2-hydroxy-3-(2-oxo-1-piperidinyl)propyl]-α-[(R)-α-(3,3-dimethyl-2-oxobutyl)hydrocinnamamido]-3-t-butoxycarbonylimidazol-4-propionamid mit Kaliumcarbonat in Methanol behandelt, wobei man (S)-N-[(1S,2S)-1-(Cyclohexylmethyl)-2-hydroxy-3-(2-oxopiperidino)propyl]-α-[(R)-α-(3,3-dimethyl-2-oxobutyl hydrocinnamamido]imidazol-4-propionamid als amorphen Festkörper erhielt, MS: 636 $(M + H)^+$.

Das als Ausgangsstoff eingesetzte (S)-N-[(1S,2S)-1-(Cyclohexylmethyl)-2-hydroxy-3-(2-oxo-1-piperidinyl)propyl]-α-[(R)-α-(3,3-dimethyl-2-oxobutyl)hydrocinnamamido]-3-t-butoxycarbonylimidazol-4-propionamid wurde, in analoger Weise wie in Beispiel 4 beschrieben, aus t-Butyl [(S)-3-amino-(S)-2-(t-butyldimethylsiloxy)-1-(cyclohexylmethyl) propyl]carbamat hergestellt, wobei jedoch anstelle des 4-Brombuttersäureäthylesters der 5-Bromvaleriansäureäthylester verwendet wurde.

## Beispiel 6

In analoger Weise wie in Beispiel 4 beschrieben, wurden die folgenden Verbindungen hergestellt:

- Aus (S)-N-[(1S,2S)-1-(Cyclohexylmethyl)-2-hydroxy-3-[methyl[3-(phenylsulfonyl)propyl]amino]propyl]-α-[(R)-α-(3,3-dimethyl-2-oxobutyl)hydrocinnamamido]-3-t-butoxycarbonylimidazol-4-propionamid das (S)-N-[(1S,-2S)-1-(Cyclohexylmethyl)-2-hydroxy-3-[methyl[3-(phenylsulfonyl)propyl]amino]propyl]-α-[(R)-α-(3,3-dimethyl-2-oxobutyl)hydrocinnamamido]imidazol-4-propionamid als amorpher Festkörper, MS: 750 $(M + H)^+$;
- aus (S)-N-[(1S,2S)-1-(Cyclohexylmethyl)-2-hydroxy-3-[methyl[6-(2-oxo-1-benzimidazolidinyl)hexyl)amino]-propyl]-α-[(R)-α-(3,3-dimethyl-2-oxobutyl)hydrocinnamamido]-3-t-butoxycarbonylimidazol-4-propionamid das (S)-N-[(1S,2S)-1-(Cyclohexylmethyl)-2-hydroxy-3-[methyl[6-(2-oxo-1-benzimidazolidinyl)hexyl]amino]propyl]-α-[(R)-α-(3,3-dimethyl-2-oxobutyl)hydrocinnamamido]imidazol-4-propionamid als amorpher Festkörper, MS: 784 $(M + H)^+$
und
- aus (S)-N-[(1S,2S)-1-(Cyclohexylmethyl)-3-[[4-(2-äthoxyäthoxy)phenäthyl]methylamino]propyl]-α-[(R)-α-(3,3-dimethyl-2-oxobutyl)hydrocinnamamido]-3-t-butoxycarbonylimidazol-4-propionamid das (S:R = 2:1)-N-[(1S,2S)-1-(Cyclohexylmethyl)-3-[[4-(2-äthoxyäthoxy)phenäthyl]methylamino]propyl]-α-[(R)-α-(3,3-dimethyl-2-oxobutyl)hydrocinnamamido]imidazol-4-propionamid-dihydrochlorid als amorpher Festkörper, MS: 760 (M +

H)$^+$. Die als Ausgangsstoffe eingesetzten Propionamide wurden wie folgt hergestellt:

(S)-N-[(1S,2S)-1-(Cyclohexylmethyl)-2-hydroxy-3[methyl-[3-(phenylsulfonyl)propyl]amino]propyl]-α-[(R)-α-(3,3-dimethyl-2-oxobutyl) hydrocinnamamido]-3-t-butoxycarbonylimidazol-4-propionamid

Diese Verbindung wurde in Analogie zu Beispiel 1 und 2 durch Umsetzen von t-Butyl [(1S,2R:S = 9:1)-1-(cyclohexylmethyl)-2,3-epoxypropyl]carbamat mit N-Methyl-3-(phenylsulfonyl)propylamin, Abspalten der Boc-Schutzgruppe mit Trifluoressigsäure (90%ig) in Methylenchlorid und Umsetzen des erhaltenen (2S,3S)-3-Amino-4-cyclohexyl-1-[methyl-[3-(phenylsulfonyl)propyl]amino]-2-butanols mit 1-(t-Butoxycarbonyl)-N-[(R)-α-(3,3-dimethyl-2-oxobutyl)hydrocinnamoyl]-L-histidin hergestellt.

(S)-N-[(1S,2S)-1-(Cyclohexylmethyl)-2-hydroxy-3-[methyl[6-(2-oxo-1-benzimidazolidinyl)hexyl]amino]propyl]-α-[(R)-α-(3,-dimethyl-2-oxobutyl)hydrocinnamamido]-3-t-butoxycarbonylimidazol-4-propionamid

Diese Verbindung wurde, ebenfalls in Analogie zu Beispiel 1 und 2, durch Umsetzen von t-Butyl [-(1S,2R:S = 9:1)-1-(cyclohexylmethyl)-2,3-epoxypropyl]carbamat mit 1-[6-(Methylamino)hexyl]-2-benzimidazolinon, Abspalten der Boc-Schutzgruppe mit 90%iger Trifluoressigsäure. in Methylenchlorid und Umsetzen des erhaltenen 1-[6-[[(2S,3S)-3-Amino-2-hydroxy-4-cyclohexylbutyl]methylamino]hexyl]-2-benzimidazolinons mit 1-(t-Butoxycarbonyl)-N-[(R)-α-(3,3-dimethyl-2-oxobutyl)hydrocinnamoyl]-L-histidin hergestellt.

(S)-N-[(1S,2S)-1-(Cyclohexylmethyl)-3-[[4-(2-äthoxyäthoxy)phenäthyl]methylamino]propyl]-α-[(R)-α-(3,3-dimethyl-2-oxobutyl)hydrocinnamamido]-3-t-butoxycarbonylimidazol-4-propionamid

Auch diese Verbindung wurde in Analogie zu Beispiel 1 und 2 durch Umsetzen von t-Butyl [-(1S,2R:S = 9:1)-1-(cyclohexylmethyl)-2,3-epoxypropyl]carbamat mit 4-(2-Aethoxyäthoxy)-N-methylphenäthylamin, Abspalten der Boc-Schutzgruppe mit 1N Salzsäure in Dioxan und Umsetzen des erhaltenen (2S,3S)-3-Amino-4-cyclohexyl-1-[[4-(2-äthoxyäthoxy)phenäthyl]methylamino]-2-butanols mit 1-(t-Butoxycarbonyl)-N-[(R)-α-(3,3-dimethyl-2-oxobutyl)hydrocinnamoyl]-L-histidin hergestellt.

Die als Ausgangsstoffe eingesetzten Amine wurden wie folgt hergestellt:

N-Methyl-3-(phenylsulfonyl)propylamin

25,5 g (71,9 mMol) 3-(Phenylsulfonyl)-1-propanol-4-methylbenzolsulfonat [vgl. H.O. Fong et al., Can. J. Chem., 57, 1206 (1979)] werden in 45 ml Methylamin gelöst und 24 Stunden bei 80° und 1000 kPa gerührt. Danach wird das Methylamin unter vermindertem Druck abgedampft, der Rückstand auf Eiswasser gegossen und mit Methylenchlorid extrahiert. Die Methylenchloridextrakte werden über Kaliumcarbonat getrocknet und unter vermindertem Druck eingedampft, wobei man 14,1 g N-Methyl-3-(phenylsulfonyl)-propylamin als Oel erhält, MS: 214 (M + H)$^+$.

4-(2-Aethoxyäthoxy)-N-methylphenäthylamin

90,12 g (1 Mol) 2-Aethocyäthanol in 1,3 l Pyridin werden bei 0° - 5° innerhalb von 15 Minuten mit 114,5 g (1 Mol) Methansulfochlorid versetzt. Das Reaktionsgemisch wird 2 Stunden bei der gleichen Temperatur gerührt und dann am Hochvakuum eingeengt. Der Rückstand wird in 1 1 Essigester aufgenommen, und die organische Lösung mit 2N Salzsäure und Wasser gewaschen, über Natriumsulfat getrocknet und eingedampft. Der so erhaltene Methansulfonsäure-2-äthoxyäthylester wird ohne weitere Reinigung in die nächste Stufe eingesetzt.

3,06 g 55%ige Natriumhydriddispersion in Oel werden mit Hexan gewaschen und mit 85 ml Dimethylformamid überschichtet. Dann werden unter Eiskühlung 17,0 g (71,6 mMol) t-Butyl [4-hydroxyphenäthyl]-carbamat [vgl. F. Rocchiccioli et al., Tetrahedron, 34, 2917 (1978)] zugegeben, und das Reaktionsgemisch 15 Minuten bei Raumtemperatur gerührt. Nach Abkühlen auf 0° wird eine Lösung von 12,04 g (71,6 mMol) Methansulfonsäure-2-äthoxyäthylester in 85 ml Dimethylformamid innerhalb von 10 Minuten zugetropft, und das Reaktionsgemisch anschliessend 24 Stunden bei Raumtemperatur gerührt. Danach wird das Reaktionsgemisch auf Eiswasser gegossen und mit Methylenchlorid extrahiert. Die Methylenchloridextrakte werden über Magnesiumsulfat getrocknet und unter vermindertem Druck eingedampft. Der erhaltene Rückstand wird an 240 g Kieselgel mit einem Gemisch von Hexan und Essigester als Eluierungsmittel chromatographiert und anschliessend aus Aether/Hexan umkristallisiert, wobei 19,1 g t-Butyl [4-(2-äthoxyäthoxy)-phenäthyl]carbamat erhalten werden, Schmelzpunkt 56°-57°.

3,2 g 55%ige Natriumhydriddispersion in Oel werden mit Hexan gewaschen und mit 190 ml Dimethylformamid überschichtet. Danach werden unter Eiskühlung und Rühren 19,0 g (61,6 mMol) t-Butyl [4-(2-äthoxyäthoxy)phenäthyl]carbamat in fester Form zugegeben, das Reaktionsgemisch 15 Minuten bei Raumtemperatur gerührt und anschliessend mit 5,7 ml Methyljodid in 10 ml Dimethylformamid innerhalb von 5 Minuten tropfenweise versetzt. Nach beendeter Zugabe wird das Reaktionsgemisch 18 Stunden bei Raumtemperatur gerührt und anschliessend auf Eiswasser gegossen und mit Methylenchlorid extrahiert. Die Methylenchloridextrakte werden über Magnesiumsulfat getrocknet und unter vermindertem Druck eingedampft, und der Rückstand an 240 g Kieselgel mit einem Gemisch von Hexan und Essigester als Eluierungsmittel chromatographiert, wobei 19,5 g t-Butyl [4-(2-äthoxyäthoxy)phenäthyl]methylcarbamat als Oel erhalten werden, welches direkt in die nächste Stufe eingesetzt wird.

17,86 g (55,4 mMol) t-Butyl [4-(2-äthoxyäthoxy)phenäthyl]methylcarbamat werden in 350 ml Dioxan gelöst, mit 66,4 ml 1N Salzsäurelösung versetzt und 2 Stunden zum Rückfluss erhitzt. Danach wird das Reaktionsgemisch eingeengt, mit Ammoniak alkalisch gestellt und mit einem 4:1-Gemisch von Methylenchlorid und Isopropanol extrahiert. Die organischen Extrakte werden über Kaliumcarbonat getrocknet und unter vermindertem Druck eingedampft, wobei 12,1 g 4-(2-Aethoxyäthoxy)-N-methylphenäthylamin als Oel erhalten werden, MS: 180 $(M - C_2H_5N)^+$.

## Beispiel 7

Ein Gemisch von 170 mg (0,74 mMol) (αS,βS)-β-Amino-α-[(isopropylamino)methyl]cyclohexylpropanol, 796 mg (1,64 mMol) 1-(t-Butoxycarbonyl)-N-[(R)-α-(3,3-dimethyl-2-oxobutyl)hydrocinnamoyl]-L-histidin, 0,46 ml (3,28 mMol) Triäthylamin, 417 mg BOPC und 20 ml Methylenchlorid wird 2 Tage bei Raumtemperatur unter Argon gerührt. Danach wird das Reaktionsgemisch zur Trockene eingedampft, der Rückstand in 20 ml Methanol gelöst, mit 20 mg Kaliumcarbonat versetzt und 2 Stunden bei Raumtemperatur gerührt. Dann wird das Reaktionsgemisch zur Trockene eingedampft, und der Rückstand mit einem 14:1:0,1-Gemisch von Methylenchlorid, Methanol und Ammoniak als Eluierungsmittel an 50 g Kieselgel chromatographiert, wobei man 80 mg (S)-N-[(1S,2S)-1-(Cyclohexylmethyl)-3-[(S)-α-[(R)-α-(3,3-dimethyl-2-oxobutyl)-hydrocinnamamido]-N-isopropylimidazol-4-propionamido]-2-hydroxypropyl]-α-[(R)-α-(3,3-dimethyl-2-oxobutyl)hydrocinnamamido]imidazol-4-propionamid als pulverförmigen Fest körper, MS: 963 $(M + H)^+$, und 270 mg (RS)-N-[(1S,2S)-1-(Cyclohexylmethyl)-2-hydroxy-3 -(isopropylamino) propyl]-α-[(R)-α-(3,3-dime thyl-2-oxobutyl)hydrocinnamamido]imidazol-4-propionamid als Schaum, MS: 596 $(M + H)^+$, erhält.

Das als Ausgangsstoff eingesetzte (αS,βS)-β-Amino-α-[(isopropylamino methyl]cyclohexylpropanol wurde wie folgt hergestellt:

Ein Gemisch von 1,0 g (3,7 mMol) t-Butyl [(1S,2R:S=9:1)-1-(cyclohexylmethyl)-2,3-epoxypropyl]-carbamat, 10 ml Methanol und 1,0 ml Isopropylamin wird 2 Tage bei Raumtemperatur gerührt. Anschliessend wird das Reaktionsgemisch zur Trockene eingedampft, der Rückstand mit 20 ml 3,6N Salzsäure in Dioxan versetzt, und das Reaktionsgemisch 4 Stunden bei Raumtemperatur stehen gelassen. Dann wird das Lösungsmittel abgedampft, und der Rückstand mit einem 50:10:1-Gemisch von Methylenchlorid, Methanol und Ammoniak als Eluierungsmittel an 50 g Kieselgel chromatographiert, wobei man 570 mg (αS,βS)-β-Amino-α-[(isopropylamino)methyl]cyclohexylpropanol als Oel erhält, MS: 229 $(M+H)^+$

In analoger Weise wie oben beschrieben wurde durch Umsetzen von t-Butyl [(1S,2R:S=9:1)-1-(cyclohexylmethyl)-2,3-epoxypropyl]carbamat mit Methylamin das [αS,βS)-β-Amino-α-[(methylamino)-methyl]cyclohexylpropanol als Oel erhalten, MS: 201 $(M + H)^+$.

## Beispiel 8

100 mg (0,168 mMol) (RS)-N-[(1S,2S)-1-(Cyclohexylmethyl)-2-hydroxy-3-(isopropylamino)propyl]-α-[(R)-α-(3,3-dimethyl-2-oxobutyl)hydrocinnamamido]imidazol-4-propionamid in 10 ml Methanol und 1 ml Triäthylamin werden mit 100 mg $(Boc)_2O$ versetzt und über Nacht bei Raumtemperatur stehen gelassen. Danach

wird das Reaktionsgemisch unter vermindertem Druck eingedampft, und der Rückstand mit einem 14:1:0,1-Gemisch von Methylenchlorid, Methanol und Ammoniak als Eluierungsmittel an 30 g Kieselgel chromatographiert. Kristallisation des so erhaltenen Rohprodukts (80 mg) aus Aether/Hexan liefert 62 mg t-Butyl [-(2S,3S)-4-cyclohexyl-3-[(R)-2-[(S)-α-(3,3-dimethyl-2-oxobutyl)hydrocinnamamido]-3-(imidazol-4-yl)-propionamido]-2-hydroxybutyl]isopropylcarbamat als weissen Festkörper, Schmelzpunkt 81°, MS: 696 $(M + H)^+$.

Beispiel 9

Ein Gemisch von 100 mg (0,168 mMol) (RS)-N-[(1S,2S)-1-(Cyclohexylmethyl)-2-hydroxy-3-(isopropylamino)propyl]-α-[(R)-α-(3,3-dimethyl-2-oxobutyl)hydrocinnamamido]imidazol-4-propionamid, 10 ml Tetrahydrofuran, 1 ml Triäthylamin und 0,065 ml (0,5 mMol) Capronsäurechlorid wird über Nacht bei Raumtemperatur stehen gelassen. Danach wird das Reaktionsgemisch unter vermindertem Druck eingedampft und dreimal mit 100 ml Essigester extrahiert. Die organischen Extrakte werden mit 2N Natriumbicarbonatlösung und gesättigter Kochsalzlösung gewaschen, getrocknet und eingedampft. Der Rückstand wird mit einem 20:1:0,1-Gemisch von Methylenchlorid, Methanol und Ammoniak als Eluierungsmittel an 30 g Kieselgel chromatographiert, wobei 57 mg (RS)-[(1S,2S)-1-(Cyclohexylmethyl)-2-hydroxy-3-N-isopropylhexanamido]-α-[(R)-α-(3,3-dimethyl-2-oxobutyl)hydrocinnamamido]imidazol-4-propionamid als Schaum erhalten werden, MS: 694 $(M + H)^+$.

Beispiel 10

In analoger Weise wie in Beispiel 7 beschrieben, wurde durch Umsetzen von [αS,βS)-β-Amino-α-[-(methylamino)methyl]cyclohexylpropanol mit 1-(t-Butoxycarbonyl)-N-[(R)-α-(3,3-dimethyl-2-oxobutyl)-hydrocinnamoyl]-L-histidin und Abspalten der Boc-Schutzgruppe mit Kaliumcarbonat in Methanol (S)-N-[-(1S,2S)-1-(Cyclohexylmethyl)-2-hydroxy-3-[[(S)-1- [(S)-α-(3,3-dimethyl-2-oxobutyl)hydrocinnamamido]-2-imidazol-4-yläthyl]methylamino]propyl]-α-[(R)-α-(3,3-dimethyl-2-oxobutyl)hydrocinnamamido]imidazol-4-propionamid in Form eines weissen Festkörpers, Schmelzpunkt 118°, MS: 935 $(M + H)^+$, als polarere Komponente, sowie (RS)-N-[(1S,2S)-1-(Cyclohexylmethyl)-2-hydroxy-3-(methylamino)propyl]-α-[(R)-α-(3,3-dimethyl-2-oxobutyl)hydrocinnamamido]imidazol-4-propionamid als weniger polare Komponente erhalten.

Beispiel 11

120 mg (0,2 mMol) (RS)-N-[(1S,2S)-1-(Cyclohexylmethyl)-2-hydroxy-3-(methylamino)propyl]-α-[(R)-α-(3,3-dimethyl-2-oxobutyl)hydrocinnamamido]imidazol-4-propionamid werden, in analoger Weise wie in Beispiel 7 beschrieben, mit 1-(t-Butoxycarbonyl)-N-(t-butoxycarbonyl)-L-histidin umgesetzt. Dann wird das Reaktionsgemisch eingedampft, und der Rückstand in 20 ml 3,6N Salzsäure in Dioxan 2 Stunden bei Raumtemperatur stehen gelassen. Danach wird das Reaktionsgemisch eingedampft, und der Rückstand in analoger Weise wie in Beispiel 9 beschrieben mit Hydrozimtsäurechlorid umgesetzt. Aufarbeiten des Reaktionsgemisches, ebenfalls in analoger Weise wie in Beispiel 9 beschrieben, liefert ein Rohprodukt, welches mit einem 140:1:0,1-Gemisch von Methylenchlorid, Methanol und Ammoniak als Eluierungsmittel an 30 g Kieselgel chromatographiert wird. Kirstallisation des so erhaltenen Rohprodukts (77 mg) aus Methylenchorid/Hexan liefert (S)-N-[(1S,2S)-1-(Cyclohexylmethyl)-2-hydroxy-3-[(S)-3-(imidazol-4-yl)-2-hydrocinnamamido-N-methylpropionamido]propyl]-α-[(R)-α-(3,3-dimethyl-2-oxobutyl)hydrocinnamamido]-imidazol-4-propionamid als weissen Festkörper, Schmelzpunkt 109°, MS: 837 $(M + H)^+$.

Beispiel 12

In analoger Weise wie in Beispiel 7 beschrieben, wurde durch Umsetzen von [αS,βS)-β-Amino-α-[-(methylamino)methyl]cyclohexylpropanol mit 1-(t-Butoxycarbonyl)-N-(t-butoxycarbonyl)-L-histidin und Abspalten der Boc-Schutzgruppe das t-Butyl [(S)-1-[[(2S,3S)-4-cyclohexyl-3-[(S)-2-(1-t-butoxyformamido)-2-imidazol-4-ylpropionamido]butyl]carbamoyl]-2-imidazol-4-yläthyl]carbamat als weisser Festkörper, MS: 675 $(M + H)^+$, und das t-Butyl [(S)-1-[[(1S,2S)-1-(cyclohexylmethyl)-2-hydroxy-3-(methylamino)propyl]-carbamoyl]-2-imidazol-4-yläthyl]carbamat als Schaum, MS: 537 $(M + H)^+$, erhalten.

## Beispiel 13

In analoger Weise wie in Beispiel 11 beschrieben, wurde durch Umsetzen von t-Butyl [(S)-1-[[(1S,2S)-1-(cyclohexylmethyl)-2-hydroxy-3-(methylamino)propyl]carbamoyl]-2-imidazol-4-yläthyl]carbamat mit Boc-Phe-OH das t-Butyl [(S)-α-[[(2S,3S)-3-[(S)-2-[(S)-α-(1-t-butoxyformamido)hydrocinnamamido]-3-imidazol-4-ylpropionamido]-4-cyclohexyl-2-hydroxybutyl]methylcarbamoyl]phenäthyl]carbamat als weisser Festkörper erhalten, MS: 832 $(M + H)^+$.

## Beispiel 14

In analoger Weise wie in Beispiel 11 beschrieben, wurde durch Abspalten der Boc-Schutzgruppe aus t-Butyl [(S)-1-[[(2S,3S)-4-cyclohexyl-3-[(S)-2-(1-t-butoxyformamido)-2-imidazol-4-ylpropionamido]butyl]-carbamoyl]-2-imidazol-4-yläthyl]carbamat und anschliessendem Umsetzen mit Boc-Phe-OH das t-Butyl [(S)-α-[[(S)-1-[[(1S,2S)-3-[[(S)-1-[(S)-α-(1-t-butoxyformamido)hydrocinnamamido]-2-imidazol-4-yläthyl]-methylcarbamoyl]-1-(cyclohexylmethyl)-2-hydroxypropyl]carbamoyl]-2-imidazol-4-yläthyl]carbamoyl]-phenäthyl]carbamat als weisser Festkörper erhalten, MS: 969 $(M + H)^+$.

## Beispiel 15

50 mg (0,08 mMol) (S)-N-[(1S,2S)-3-Amino-1-(cyclohexylmethyl)-2-hydroxypropyl]-α-[(R)-α-(3,3-dimethyl-2-oxobutyl)hydrocinnamamido]imidazol-4-propionamid-hydrochlorid und 1,5 g (10 mMol) Phthalsäureanhydrid werden in 15 ml Acetonitril suspendiert und über Nacht zum Rückfluss erhitzt. Danach wird das Reaktionsgemisch unter vermindertem Druck zur Trockene eingedampft, und der Rückstand mit einem Gemisch von Methylenchlorid, Methanol und Ammoniak als Eluierungsmittel an 10 g Kieselgel chromatographiert, wobei 31 mg (S)-N-[(1S,2S)-1-(Cyclohexylmethyl)-2-hydroxy-3-phthalimidopropyl]-α-[(R)-α-(3,3-dimethyl-2-oxobutyl)hydrocinnamamido]imidazol-4-propionamid als Schaum erhalten werden, MS: 684 $(M + H)^+$.

## Beispiel 16

In analoger Weise wie in Beispiel 4 beschrieben, wurden durch Abspalten der Boc-Schutzgruppe mit Kaliumcarbonat in Methanol aus (S)-N-[(1S,2RS)-1-(Cyclohexylmethyl)-2-hydroxy-3-nitropropyl]-α-[(R)-α-(3,3-dimethyl-2-oxobutyl)hydrocinnamamido]-3-t-butoxycarbonylimidazol-4-propionamid und anschliessender chromatographischer Reinigung an 50 g Kieselgel unter Verwendung eines 200:10:1-Gemisches von Methylenchlorid, Methanol und Ammoniak als Eluierungsmittel die beiden epimeren Verbindungen (S)-N-[(1S,2S oder R)-1-(Cyclohexylmethyl)-2-hydroxy-3-nitropropyl]-α-[(R)-α-(3,3-dimethyl-2-oxobutyl) hydrocinnamamido] imidazol-4-propionamid und (S)-N-[(1S,2R oder S)-1-(Cyclohexylmethyl)-2-hydroxy-3-nitropropyl]-α-[(R)-α-(3,3-dimethyl-2-oxobutyl)hydrocinnamamido]imidazol-4-propionamid, beide jeweils als Schaum erhalten, MS (jeweils): 584 $(M + H)^+$.

Das als Ausgangsstoff eingesetzte (S)-N-[(1S,2RS)-1-(Cyclohexylmethyl)-2-hydroxy-3-nitropropyl]-α-[(R)-α-(3,3-dimethyl-2-oxobutyl)hydrocinnamamido]-3-t-butoxycarbonyl imidazol-4-propionamid wurde wie folgt hergestellt:

Zu 0,40 ml (7,5 mMol) Nitromethan in 5 ml Tetrahydrofuran werden 306,8 mg 65%ige Natriumhydriddispersion in Oel gegeben, und das Reaktionsgemisch eine Stunde bei 50° gehalten. Danach wird das Reaktionsgemisch auf -78° abgekühlt und tropfenweise mit einer Lösung von 383 mg (1,5 mMol) 2-t-Butoxycarbonylamino-3(S)-cyclohexylpropylaldehyd in 5 ml Tetrahydrofuran versetzt. Danach wird das Kühlbad entfernt, das Gemisch noch 3 Stunden stehen gelassen, dann auf Eis gegossen und dreimal mit 120 ml Essigester extrahiert. Die Essigesterextrakte werden mit 60 ml gesättigter Kochsalzlösung gewaschen, über Magnesiumsulfat getrocknet und eingedampft. Chromatographie des erhaltenen Rohprodukts (390 mg) an 35 g Kieselgel unter Verwendung eines 95:5-Gemisches von Toluol und Essigester als Eluierungsmittel liefert 170 mg t-Butyl [(1S,2RS)-1-(cyclohexylmethyl)-3-hydroxy-3-nitropropyl]carbamat als Oel, MS: 317 $(M + H)^+$.

In analoger Weise wie in Beispiel 7 beschrieben, wurde durch Abspalten der Boc-Schutzgruppe mit Salzsäure in Dioxan aus t-Butyl [(1S,2RS)-1-(cyclohexylmethyl)-2-hydroxy-3-nitropropyl]carbamat und Umsetzen des intermediär erhaltenen [(1S,2RS)-2-Amino-1-(cyclohexylmethyl)-4-nitrobutan-3-ols mit 1-(t-Butoxycarbonyl)-N-[(R)-α-(3,3-dimethyl-2-oxobutyl)hydrocinnamoyl]-L-histidin das (S)-N-[(1S,2RS)-1-(Cyclohexylmethyl)-2-hydroxy-3-nitropropyl]-α-[(R)-α-(3,3-dimethyl-2-oxobutyl)hydrocinnamamido]-3-t-butoxycarbonylimidazol-4-propionamid erhalten, das direkt in die nächste Stufe eingesetzt wurde.

## Beispiel A

Eine sterilfiltrierte wässrige Lösung von t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]-carbamoyl]-2-imidazol-4-yläthyl]carbamoyl]phenäthyl]carbamat wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]carbamoyl]-2-imidazol-4-yläthyl]carbamoyl]phenäthyl]carbamat | 3,0 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

## Beispiel B

Man löst 5 mg t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]carbamoyl]-2-imidazol-4-yläthyl]carbamoyl]phenäthyl]carbamat in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel C

In einer Mischung von 3,5 ml Myglyol 812 und 0,08 g Benzylalkohol werden 500 mg fein gemahlenes (5,0 µm) t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]carbamoyl]-2-imidazol-4-yläthyl]-carbamoyl]phenäthyl]carbamat suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

Beispiel D

Wenn man nach den in den Beispielen A-C beschriebenen Verfahren arbeitet, können aus den folgenden, ebenfalls bevorzugten Verbindungen entsprechende galenische Präparate hergestellt werden:
(S)-N-[(1S,2S)-1-(Cyclohexylmethyl)-2-hydroxy-3-(2-oxopiperidino)propyl]-α-[(R)-α-(3,3-dimethyl-2-oxobutyl)-hydrocinnamamido]imidazol-4-propionamid;
(S)-N-[(1S,2S)-1-(Cyclohexylmethyl)-3-[(S)-α-[(R)-α-(3,3-dimethyl-2-oxobutyl)hydrocinnamamido]-N-isopropylimidazol-4-propionamido]-2-hydroxypropyl]-α-[(R)-α-(3,3-dimethyl-2-oxobutyl)hydrocinnamamido]-imidazol-4-propionamid;
(S)-N-[(1S,2S)-1-(Cyclohexylmethyl)-2-hydroxy-3-[[(S)-1-[(S)-α-(3,3-dimethyl-2-oxobutyl)hydrocinnamamido]-2-imidazol-4-yläthyl]methylamino]propyl]-α-[(R)-α-(3,3-dimethyl-2-oxobutyl)hydrocinnamamido]imidazol-4-propionamid;
(S)-N-[(1S,2S)-1-(Cyclohexylmethyl)-2-hydroxy-3-[(S)-3-(imidazol-4-yl)-2-hydrocinnamamido-N-methylpropionamido]propyl]-α-[(R)-α-(3,3-dimethyl-2-oxobutyl)hydrocinnamamido]imidazol-4-propionamid;
t-Butyl [(S)-α-[[(2S,3S)-3-[(S)-2-[(S)-α-(1-t-butoxyformamido)hydrocinnamamido]-3-imidazol-4-ylpropionamido]-4-cyclohexyl-2-hydroxybutyl]methylcarbamoyl]phenäthyl]carbamat und
(S)-N-[(1S,2S)-1-(Cyclohexylmethyl)-2-hydroxy-3-phthalimidopropyl]-α-[(R)-α-(3,3-dimethyl-2-oxobutyl)-hydrocinnamamido]imidazol-4-propionamid.

**Ansprüche**

1. Aminosäurederivate der allgemeinen Formel

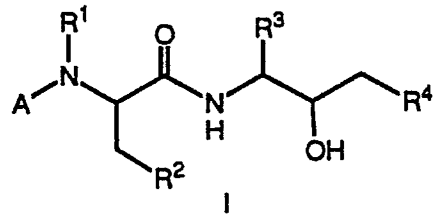

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Nitro, Amino oder eine Gruppe der Formel-N($R^5$)($R^6$) und A eine der Gruppen

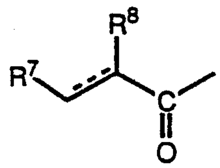

(a)

und -Y-Z (b)

bedeuten, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl, gegebenenfalls substituiertes Benzimidazolonyl oder den Rest einer gegebenenfalls acylierten Aminosäure oder eines gegebenenfalls acylierten Dipeptids oder $R^5$ und $R^6$ zusammen mit dem sie verbindenden Stickstoffatom einen 5- oder 6-gliedrigen Heterocyclus, ein 5- oder 6-gliedriges Lactam oder ein 5- oder 6-gliedriges Imid bedeuten, mit der Massgabe, dass A nicht die Gruppe (b) bedeuten kann, wenn $R^6$ Alkanoyl, Alkoxycarbonyl oder Arylalkoxy carbonyl bedeutet, die gestrichelte Linie eine zusätzliche Bindung bedeuten kann, $R^7$ Phenyl, substituiertes Phenyl, Benzyl oder Naphthyl und $R^8$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^8$ nicht Alkoxycarbonylamino oder Arylalkoxycarbonylamino bedeuten kann, wenn $R^7$ Phenyl, Benzyl oder $\alpha$-Naphthyl bedeutet, Y den N-terminal mit Z verbundenen, bivalenten Rest von gegebenenfalls N- und/oder $\alpha$-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, $\alpha$-Naphthylalanin oder Homophenylalanin und Z Wasserstoff oder Acyl bedeuten,

in Form von optisch reinen Diastereomeren, Diastereomerengemischen, diastereomeren Racematen oder Gemischen von diastereomeren Racematen sowie pharmazeutisch verwendbare Salze dieser Verbindungen.

2. Verbindungen gemäss Anspruch 1, worin $R^1$ Wasserstoff bedeutet.

3. Verbindungen gemäss Anspruch 1 oder 2, worin $R^2$ Imidazol-2-yl, Imidazol-4-yl oder Thiazol-4-yl, vorzugsweise Imidazol-4-yl, bedeutet.

4. Verbindungen gemäss einem der Ansprüche 1-3, worin $R^3$ Cyclohexylmethyl bedeutet.

5. Verbindungen gemäss einem der Ansprüche 1-4, worin $R^4$ die Gruppe-$N(R^5)(R^6)$ bedeutet.

6. Verbindungen gemäss Anspruch 5, worin $R^5$ Alkyl, vorzugsweise Methyl, und $R^6$ den Rest einer gegebenenfalls acylierten Aminosäure oder eines gegebenenfalls acylierten Dipeptids, vorzugsweise den acylierten Rest von Histidin oder Phenylalanin oder den acylierten Rest des Dipeptids aus Histidin und Phenylalanin, oder $R^5$ und $R^6$ zusammen mit dem sie verbindenden Stickstoffatom ein 5- oder 6-gliedriges Lactam bedeuten..

7. Verbindungen gemäss einem der Ansprüche 1-6, worin A die Gruppe (a) bedeutet.

8. Verbindungen gemäss einem der Ansprüche 1-7, worin $R^7$ Phenyl oder substituiertes Phenyl, vorzugsweise Phenyl, bedeutet.

9. Verbindungen gemäss einem der Ansprüche 1-8, worin $R^8$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, vorzugsweise Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeutet.

10. Verbindungen gemäss einem der Ansprüche 1-6, worin A die Gruppe (b) bedeutet, worin Y den N-terminal mit Z verbundenen, bivalenten Rest von Phenylalanin bedeutet.

11. Verbindungen gemäss einem der Ansprüche 1-6 und 10, worin Z die Grupe $R^a$-O-CO- bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, vorzugsweise einen gesättigten, aliphatishen Kohlenwasserstoffrest mit bis zu 6 Kohlenstoffatomen oder einen heteroaromatischen Rest mit bis zu 10 Kohlenstoffatomen, bedeutet.

12. Verbindungen gemäss einem der Ansprüche 1-9, worin $R^1$ Wasserstoff, $R^2$ Imidazol-4-yl, $R^3$

Cyclohexylmethyl, $R^4$ die Gruppe -N($R^5$)($R^6$), $R^5$ Methyl, $R^6$ den acylierten Rest von Histidin oder Phenylalanin oder den acylierten Rest des Dipeptids aus Histidin und Phenylalanin, $R^7$ Phenyl und $R^8$ $C_1$-$C_4$-Alkylcarbonylmethyl oder $C_1$-$C_4$-Alkylsulfonylmethyl bedeuten.

13. (S)-N-[(1S,2S)-1-(Cyclohexylmethyl)-2-hydroxy-3-(2-oxopiperidino)propyl]-α-[(R)-α-(3,3-dimethyl-2-oxobutyl)hydrocinnamamido]imidazol-4-propionamid, (S)-N-[(1S,2S)-1-(Cyclohexylmethyl)-3-[(S)-α-[(R)-α-(3,3-dimethyl-2-oxobutyl)hydrocinnamamido]-N-isopropylimidazol-4-propionamid]-2-hydroxypropyl]-α-[(R)-α-(3,3-dimethyl-2-oxobutyl)hydrocinnamamido]imidazol-4-propionamid, (S)-N-[(1S,2S)-1-(Cyclohexylmethyl)-2-hydroxy-3-[[(S)-1-[(S)-α-(3,3-dimethyl-2-oxobutyl)hydrocinnamamido]-2-imidazol-4-yläthyl]methylamino]-propyl]-α-[(R)-α-(3,3-dimethyl-2-oxobutyl)hydrocinnamamido]imidazol-4- propionamid, (S)-N-[(1S,2S)-1-(Cyclohexylmethyl)-2-hydroxy-3-[(S)-3-(imidazol-4-yl)-2-hydrocinnamamido-N-methylpropionamido]propyl]-α-[(R)-α-(3,3-dimethyl-2-oxobutyl)hydrocinnamamido]imidazol-4-propionamid, t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]carbamoyl]-2-imidazol]-4-yläthyl]carbamoyl]phenäthyl]carbamat, t-Butyl [(S)-α--[[(2S,3S)-3-[(S)-2-[(S)-α-(1-t-butoxyformamido)hydrocinnamamido]-3-imidazol-4-ylpropionamidol]-4-cyclohexyl-2-hydroxybutyl]methylcarbamoyl]phenäthyl]carbamat oder (S)-N-[(1S,2S)-1-(Cyclohexylmethyl)-2-hydroxy-3-phthalimidopropyl]-α-[(R)-α-(3,3-dimethyl-2-oxobutyl)hydrocinnamamido]imidazol-4-propionamid.

14. Verbindungen der allgemeinen Formel

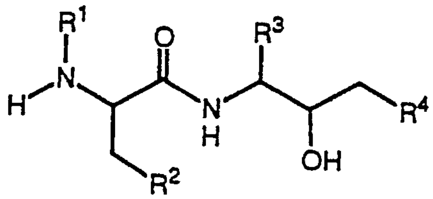

II

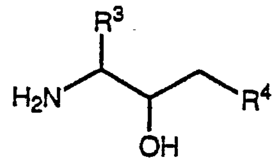

III

und

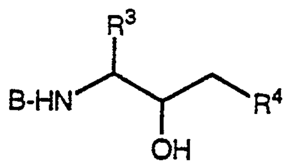

V

worin B eine Aminoschutzgruppe, vorzugsweise t-Butoxycarbonyl oder Benzyloxycarbonyl, $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Nitro, Amino oder eine Gruppe der Formel -N($R^5$)($R^6$) bedeuten, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl, gegebenenfalls substituiertes Benzimidazolonyl oder den Rest einer gegebenenfalls acylierten Aminosäure oder eines gegebenenfalls acylierten Dipeptids oder $R^5$ und $R^6$ zusammen mit dem sie verbindenden Stickstoffatom einen 5- oder 6-gliedrigen Heterocyclus, ein 5- oder 6-gliedriges Lactam oder ein 5- oder 6-gliedriges Imid bedeuten.

15. Aminosäurederivate gemäss einem der Ansprüche 1-13 zur Anwendung als therapeutische Wirkstoffe.

16. Aminosäurederivate gemäss einem der Ansprüche 1-13 zur Anwendung bei der Bekämpfung bzw. Verhütung von Bluthochdruck und Herzinsuffizienz.

17. Verfahren zur Herstellung einer Verbindung gemäss einem der Ansprüche 1-13, dadurch gekennzeichnet, dass man

a) eine Verbindung der allgemeinen Formel

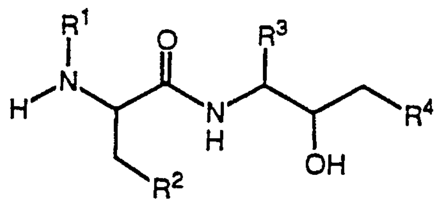

II

worin $R^1$, $R^2$, $R^3$ und $R^4$ die in Anspruch 1 angegebene Bedeutung besitzen, mit einem die Gruppe

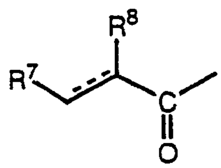 (a) oder -Y-Z (b)

worin $R^7$, $R^8$, Y, Z und die gestrichelte Linie die in Anspruch 1 angegebene Bedeutung besitzen, abgebenden Acylierungsmittel umsetzt, oder

b) eine Verbindung der allgemeinen Formel

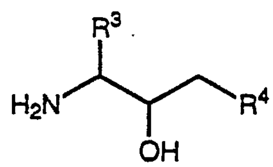

III

worin $R^3$ und $R^4$ die in Anspruch 1 angegebene Bedeutung besitzen, mit einer Verbindung der allgemeinen Formel

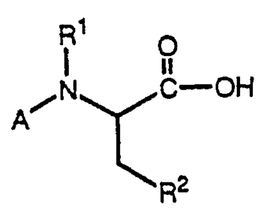

IV

worin $R^1$, $R^2$ und A die in Anspruch 1 angegebene Bedeutung besitzen, oder einem aktivierten Derivat davon umsetzt, oder

c) eine Verbindung der Formel I, worin Z Wasserstoff bedeutet und die übrigen Symbole die in Anspruch 1 angegebene Bedeutung besitzen, mit einer gegebenenfalls acylierten Aminosäure oder einem gegebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeutet, aus einer entsprechenden Verbindung der Formel I, worin A eine N-geschützte Aminogruppe enthält, und/oder aus einer der Formel I entsprechenden Verbindung, worin $R^4$ jedoch N-gechütztes Amino und/oder $R^2$ N-geschütztes Imidazol-2-yl, Imidazol-4-yl oder Pyrazol-3-yl bedeutet, die N-Schutzgruppe(n) abspaltet, oder

e) zur Herstellung einer Verbindung der Formel I, worin $R^4$ ein 5- oder 6-gliedriges Imid bedeutet,

eine entsprechende Verbindung der Formel I, worin $R^4$ Amino bedeutet, mit einem Anhydrid einer zweibasischen Säure umsetzt, oder

f) zur Herstellung einer Verbindung der Formel I, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl oder den Rest einer gegebenenfalls acylierten Aminosäure oder eines gegebenenfalls acylierten Dipeptids bedeuten, eine der Formel I entsprechende Verbindung, worin $R^6$ jedoch Wasserstoff und $R^5$ Alkyl, Alkoxyalkyl oder gegebenenfalls substituiertes Phenyl, Phenylalkyl oder Phenylsulfonylalkyl bedeuten, mit einem den Rest $R^6$ abgebenden Acylierungsmittel umsetzt, und

g) erwünschtenfalls ein Gemisch diastereomerer Racemate in die diastereomeren Racemate oder optisch reinen Diastereomeren auftrennt, und/oder

h) erwünschtenfalls ein Diastereomerengemisch in die optisch reinen Diastereomeren auftrennt, und/oder

i) erwünschtenfalls eine erhaltene Verbindung in ein pharmazeutisch verwendbares Salz überführt.

18. Arzneimittel, enthaltend ein Aminosäurederivat gemäss einem der Ansprüche 1-13 und ein therapeutisch inertes Excipiens.

19. Mittel zur Bekämpfung bzw. Verhütung von Bluthochdruck und Herzinsuffizienz, enthaltend ein Aminosäurederivat gemäss einem der Ansprüche 1-13 und ein therapeutisch inertes Excipiens.

20. Verwendung eines Aminosäurederivates gemäss einem der Ansprüche 1-13 bei der Bekämpfung bzw. Verhütung von Krankheiten.

21. Verwendung eines Aminosäurederivates gemäss einem der Ansprüche 1-13 bei der Bekämpfung bzw. Verhütung von Bluthochdruck und Herzinsuffizienz.

22. Verwendung eines Aminosäurederivates gemäss einem der Ansprüche 1-13 zur Herstellung von Mitteln gegen Bluthochdruck und/oder Herzinsuffizienz.

Patentansprüche für folgenden Vertragsstaat: ES

1. Verfahren zur Herstellung von Aminosäurederivaten der allgemeinen Formel

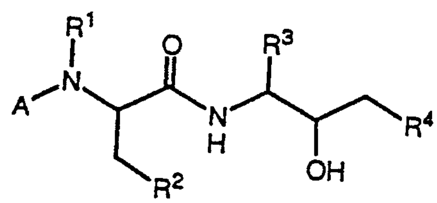

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Nitro, Amino oder eine Gruppe der Formel $-N(R^5)(R^6)$ und A eine der Gruppen

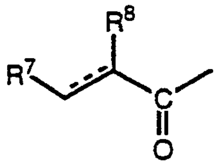

(a) und -Y-Z (b)

bedeuten, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl, gegebenenfalls substituiertes Benzimidazolonyl oder den Rest einer gegebenenfalls acylierten Aminosäure oder eines gegebenenfalls acylierten Dipeptids oder $R^5$ und $R^6$ zusammen mit dem sie verbindenden Stickstoffatom einen 5- oder 6-gliedrigen Heterocyclus, ein 5- oder 6-gliedriges Lactam oder ein 5- oder 6-gliedriges Imid bedeuten, mit der Massgabe, dass A nicht die Gruppe (b) bedeuten kann, wenn $R^6$ Alkanoyl, Alkoxycarbonyl oder Arylalkoxycarbonyl bedeutet,

die gestrichelte Linie eine zusätzliche Bindung bedeuten kann, $R^7$ Phenyl, substituiertes Phenyl, Benzyl oder Naphthyl und $R^8$ Wasserstoff, Alkoxycarbonylalkyl, Alkylcarbonylalkyl, Cycloalkylcarbonylalkyl, Heterocycloalkylcarbonylalkyl, Arylcarbonylalkyl. Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl. Dialkoxyphosphoroxyalkyl, Diphenyloxyphosphoroxyalkyl. Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^8$ nicht Alkoxycarbonylamino oder Arylalkoxycarbonylamino bedeuten kann, wenn $R^7$ Phenyl, Benzyl oder α-Naphthyl bedeutet, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin und Z Wasserstoff oder Acyl bedeuten,

in Form von optisch reinen Diastereomeren, Diastereomerengemischen, diastereomeren Racematen oder Gemischen von diastereomeren Racematen sowie von pharmazeutisch verwendbaren Salzen dieser Verbindungen, dadurch gekennzeichnet, dass man

a) eine Verbindung der allgemeinen Formel

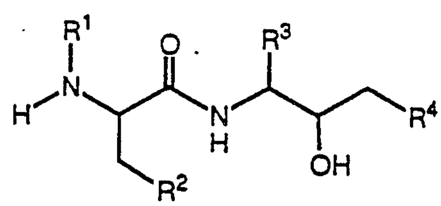

II

worin $R^1$, $R^2$, $R^3$ und $R^4$ die oben angegebene Bedeutung besitzen, mit einem die Gruppe

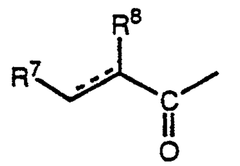 (a) oder -Y-Z (b)

worin $R^7$, $R^8$, Y, Z und die gestrichelte Linie die oben angegebene Bedeutung besitzen, abgebenden Acylierungsmittel umsetzt, oder

b) eine Verbindung der allgemeinen Formel

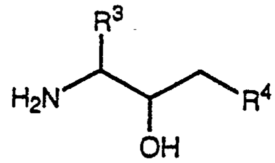

III

worin $R^3$ und $R^4$ die oben angegebene Bedeutung besitzen, mit einer Verbindung der allgemeinen Formel

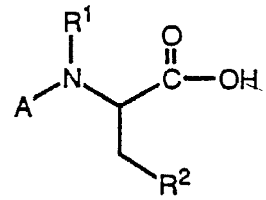

IV

worin $R^1$, $R^2$ und A die oben angegebene Bedeutung besitzen, oder einem aktivierten Derivat davon umsetzt, oder

c) eine Verbindung der Formel I, worin Z Wasserstoff bedeutet und die übrigen Symbole die oben angegebene Bedeutung besitzen, mit einer gegebenenfalls acylierten Aminosäure oder einem gegebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeutet, aus einer entsprechenden Verbindung der Formel I, worin A eine N-geschützte Aminogruppe enthält, und/oder aus einer der Formel I entsprechenden Verbindung, worin $R^4$ jedoch N-gechütztes Amino und/oder $R^2$ N-geschütztes Imidazol-2-yl, Imidazol-4-yl oder Pyrazol-3-yl bedeutet, die N-Schutzgruppe(n) abspaltet, oder

e) zur Herstellung einer Verbindung der Formel I, worin $R^4$ ein 5- oder 6-gliedriges Imid bedeutet, eine entsprechende Verbindung der Formel I, worin $R^4$ Amino bedeutet, mit einem Anhydrid einer zweibasischen Säure umsetzt, oder

f) zur Herstellung einer Verbindung der Formel I, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl oder den Rest einer gegebenenfalls acylierten Aminosäure oder eines gegebenenfalls acylierten Dipeptids bedeuten, eine der Formel I entsprechende Verbindung, worin $R^6$ jedoch Wasserstoff und $R^5$ Alkyl, Alkoxyalkyl oder gegebenenfalls substituiertes Phenyl, Phenylalkyl oder Phenylsulfonylalkyl bedeuten, mit einem den Rest $R^6$ abgebenden Acylierungsmittel umsetzt, und

g) erwünschtenfalls ein Gemisch diastereomerer Racemate in die diastereomeren Racemate oder optisch reinen Diastereomeren auftrennt, und/oder

h) erwünschtenfalls ein Diastereomerengemisch in die optisch reinen Diastereomeren auftrennt, und/oder

i) erwünschtenfalls eine erhaltene Verbindung in ein pharmazeutisch verwendbares Salz überführt.

2. Verfahren gemäss Anspruch 1, worin $R^1$ Wasserstoff bedeutet.

3. Verfahren gemäss Anspruch 1 oder 2, worin $R^2$ Imidazol-2-yl, Imidazol-4-yl oder Thiazol-4-yl, vorzugsweise Imidazol-4-yl, bedeutet.

4. Verfahren gemäss einem der Ansprüche 1-3, worin $R^3$ Cyclohexylmethyl bedeutet.

5. Verfahren gemäss einem der Ansprüche 1-4, worin $R^4$ die Gruppe -N($R^5$)($R^6$) bedeutet.

6. Verfahren gemäss Anspruch 5, worin $R^5$ Alkyl, vorzugsweise Methyl, und $R^6$ den Rest einer gegebenenfalls acylierten Aminosäure oder eines gegebenenfalls acylierten Dipeptids, vorzugsweise den acylierten Rest von Histidin oder Phenylalanin oder den acylierten Rest des Dipeptids aus Histidin und Phenylalanin, oder $R^5$ und $R^6$ zusammen mit dem sie verbindenden Stickstoffatom ein 5- oder 6-gliedriges Lactam bedeuten.

7. Verfahren gemäss einem der Ansprüche 1-6, worin A die Gruppe (a) bedeutet.

8. Verfahren gemäss einem der Ansprüche 1-7, worin $R^7$ Phenyl oder substituiertes Phenyl, vorzugsweise Phenyl, bedeutet.

9. Verfahren gemäss einem der Ansprüche 1-8, worin $R^8$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, vorzugsweise Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeutet.

10. Verfahren gemäss einem der Ansprüche 1-6, worin A die Gruppe (b) bedeutet, worin Y den N-terminal mit Z verbundenen, bivalenten Rest von Phenylalanin bedeutet.

11. Verfahren gemäss einem der Ansprüche 1-6 und 10, worin Z die Grupe $R^a$-O-CO- bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit

bis zu 18 Kohlenstoffatomen, vorzugsweise einen gesättigten, aliphatishen Kohlenwasserstoff rest mit bis zu 6 Kohlenstoffatomen oder einen heteroaromatischen Rest mit bis zu 10 Kohlenstoffatomen, bedeutet.

12. Verfahren gemäss einem der Ansprüche 1-9, worin $R^1$ Wasserstoff, $R^2$ Imidazol-4-yl, $R^3$ Cyclohexylmethyl, $R^4$ die Gruppe $-N(R^5)(R^6)$, $R^5$ Methyl, $R^6$ den acylierten Rest von Histidin oder Phenylalanin oder den acylierten Rest des Dipeptids aus Histidin und Phenylalanin, $R^7$ Phenyl und $R^8$ oder $C_1$-$C_4$-Alkylcarbonylmethyl oder $C_1$-$C_4$-Alkylsulfonylmethyl bedeuten.

13. Verfahren gemäss Anspruch 1, dadurch gekennzeichnet, dass man (S)-N-[(1S,2S)-1-(Cyclohexylmethyl)-2-hydroxy-3-(2-oxopiperidino)propyl]-α-[(R)-α-(3,3-dimethyl-2-oxobutyl)-hydrocinnamamido]imidazol-4-propionamid herstellt.

14. Verfahren gemäss Anspruch 1, dadurch gekennzeichnet, dass man (S)-N-[(1S,2S)-1-(Cyclohexylmethyl)-3-[(S)-α-[(R)-α-(3,3-dimethyl-2-oxobutyl)hydrocinnamamido]-N-isopropylimidazol-4-propionamido]-2-hydroxypropyl]-α-[(R)-α-(3,3-dimethyl-2-oxobutyl)hydrocinnamamido]imidazol-4-propionamid herstellt.

15. Verfahren gemäss Anspruch 1, dadurch gekennzeichnet, dass man (S)-N-[(1S,2S)-1-(Cyclohexylmethyl)-2-hydroxy-3-[[(S)-1-[(S)-α-(3,3-dimethyl-2-oxobutyl)hydrocinnamamido]-2-imidazol-4-yläthyl]methylamino]propyl]-α-[(R)-α-(3,3-dimethyl-2-oxobutyl)hydrocinnamamido]imidazol-4-propionamid herstellt.

16. Verfahren gemäss Anspruch 1, dadurch gekennzeichnet, dass man (S)-N-[(1S,2S)-1-(Cyclohexylmethyl)-2-hydroxy-3-[(S)-3-(imidazol-4-yl)-2-hydrocinnamamido-N-methylpropionamido]propyl]-α-[(R)-α-(3,3-dimethyl-2-oxobutyl)hydrocinnamamido]imidazol-4-propionamid herstellt.

17. Verfahren gemäss Anspruch 1, dadurch gekennzeichnet, dass man t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]carbamoyl]-2-imidazol-4-yl-äthyl]carbamoyl]phenäthyl]carbamat herstellt.

18. Verfahren gemäss Anspruch 1, dadurch gekennzeichnet, dass man t-Butyl [(S)-α-[[(2S,3S)-3-[(S)-2-[(S)-α-(1-t-butoxyformamido)hydrocinnamamido]-3-imidazol-4-ylpropionamido]-4-cyclohexyl-2-hydroxybutyl]-methylcarbamoyl]phenäthyl]carbamat herstellt.

19. Verfahren gemäss Anspruch 1, dadurch gekennzeichnet, dass man (S)-N-[(1S,2S)-1-(Cyclohexylmethyl)-2-hydroxy-3-phthalimidopropyl]-α-[(R)-α-(3,3-dimethyl-2-oxobutyl)hydrocinnamamido]-imidazol-4-propionamid herstellt.

20. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

21. Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

Patentansprüche für folgenden Vertragsstaat: GR

1. Verfahren zur Herstellung von Aminosäurederivaten der allgemeinen Formel

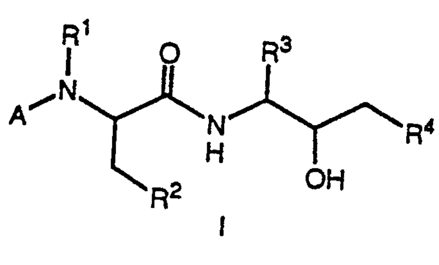

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Nitro, Amino oder eine Gruppe der Formel $-N(R^5)(R^6)$ und A eine der Gruppen

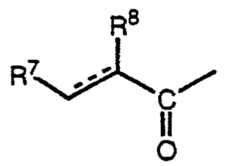 (a) und -Y-Z (b)

bedeuten, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl, gegebenenfalls substituiertes Benzimidazolonyl oder den Rest einer gegebenenfalls acylierten Aminosäure oder eines gegebenenfalls acylierten Dipeptids oder $R^5$ und $R^6$ zusammen mit dem sie verbindenden Stickstoffatom einen 5- oder 6-gliedrigen Heterocyclus, ein 5- oder 6-gliedriges Lactam oder ein 5- oder 6-gliedriges Imid bedeuten, mit der Massgabe, dass A nicht die Gruppe (b) bedeuten kann, wenn $R^6$ Alkanoyl, Alkoxycarbonyl oder Arylalkoxycarbonyl bedeutet, die gestrichelte Linie eine zusätzliche Bindung bedeuten kann, $R^7$ Phenyl, substituiertes Phenyl, Benzyl oder Naphthyl und $R^8$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Aminoalkylcarbonylalkyl, substituiertes Aminoalkylcarbonylalkyl, Aminoalkylsulfonylalkyl, substituiertes Aminoalkylsulfonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl, Alkylsulfonylalkyl, Arylthioalkyl, Arylsulfinylalkyl, Arylsulfonylalkyl, Arylalkylthioalkyl, Arylalkylsulfinylalkyl oder Arylalkylsulfonylalkyl bedeuten, mit der Massgabe, dass $R^8$ nicht Alkoxycarbonylamino oder Arylalkoxycarbonylamino bedeuten kann, wenn $R^7$ Phenyl, Benzyl oder α-Naphthyl bedeutet, Y den N-terminal mit Z verbundenen, bivalenten Rest von gegebenenfalls N- und/oder α-methyliertem Phenylglycin, Cyclohexylglycin, Phenylalanin, Cyclohexylalanin, 4-Fluorphenylalanin, 4-Chlorphenylalanin, Tyrosin, O-Methyltyrosin, α-Naphthylalanin oder Homophenylalanin und Z Wasserstoff oder Acyl bedeuten,

in Form von optisch reinen Diastereomeren, Diastereomerengemischen, diastereomeren Racematen oder Gemischen von diastereomeren Racematen sowie von pharmazeutisch verwendbaren Salzen dieser Verbindungen, dadurch gekennzeichnet, dass man

a) eine Verbindung der allgemeinen Formel

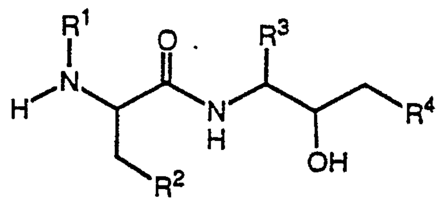

II

worin $R^1$, $R^2$, $R^3$ und $R^4$ die oben angegebene Bedeutung besitzen,
mit einem die Gruppe

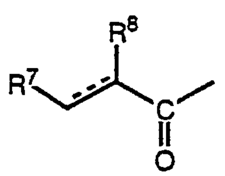 (a) oder -Y-Z (b)

worin $R^7$, $R^8$, Y, Z und die gestrichelte Linie die oben angegebene Bedeutung besitzen,
abgebenden Acylierungsmittel umsetzt, oder

b) eine Verbindung der allgemeinen Formel

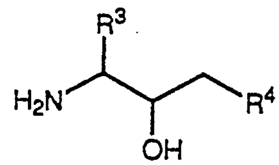

III

worin $R^3$ und $R^4$ die oben angegebene Bedeutung besitzen, mit einer Verbindung der allgemeinen Formel

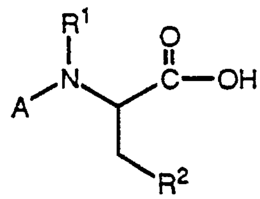

IV

worin $R^1$, $R^2$ und A die oben angegebene Bedeutung besitzen, oder einem aktivierten Derivat davon umsetzt, oder

c) eine Verbindung der Formel I, worin Z Wasserstoff bedeutet und die übrigen Symbole die oben angegebene Bedeutung besitzen, mit einer gegebenenfalls acylierten Aminosäure oder einem gegebenenfalls acylierten Dipeptid umsetzt, oder

d) zur Herstellung einer Verbindung der Formel I, worin A eine freie Aminogruppe enthält und/oder $R^4$ Amino und/oder $R^2$ Imidazol-2-yl, Imidazol-4-yl oder Pyrazol-3-yl bedeutet, aus einer entsprechenden Verbindung der Formel I, worin A eine N-geschützte Aminogruppe enthält, und/oder aus einer der Formel I entsprechenden Verbindung, worin $R^4$ jedoch N-gechütztes Amino und/oder $R^2$ N-geschütztes Imidazol-2-yl, Imidazol-4-yl oder Pyrazol-3-yl bedeutet, die N-Schutzgruppe(n) abspaltet, oder

e) zur Herstellung einer Verbindung der Formel I, worin $R^4$ ein 5- oder 6-gliedriges Imid bedeutet, eine entsprechende Verbindung der Formel I, worin $R^4$ Amino bedeutet, mit einem Anhydrid einer zweibasischen Säure umsetzt, oder

f) zur Herstellung einer Verbindung der Formel I, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl oder den Rest einer gegebenenfalls acylierten Aminosäure oder eines gegebenenfalls acylierten Dipeptids bedeuten, eine der Formel I entsprechende Verbindung, worin $R^6$ jedoch Wasserstoff und $R^5$ Alkyl, Alkoxyalkyl oder gegebenenfalls substituiertes Phenyl, Phenylalkyl oder Phenylsulfonylalkyl bedeuten, mit einem den Rest $R^6$ abgebenden Acylierungsmittel umsetzt, und

g) erwünschtenfalls ein Gemisch diastereomerer Racemate in die diastereomeren Racemate oder optisch reinen Diastereomeren auftrennt, und/oder

h) erwünschtenfalls ein Diastereomerengemisch in die optisch reinen Diastereomeren auftrennt, und/oder

i) erwünschtenfalls eine erhaltene Verbindung in ein pharmazeutisch verwendbares Salz überführt.

2. Verfahren gemäss Anspruch 1, worin $R^1$ Wasserstoff bedeutet.

3. Verfahren gemäss Anspruch 1 oder 2, worin $R^2$ Imidazol-2-yl, Imidazol-4-yl oder Thiazol-4-yl, vorzugsweise Imidazol-4-yl, bedeutet.

4. Verfahren gemäss einem der Ansprüche 1-3, worin $R^3$ Cyclohexylmethyl bedeutet.

5. Verfahren gemäss einem der Ansprüche 1-4, worin $R^4$ die Gruppe $-N(R^5)(R^6)$ bedeutet.

6. Verfahren gemäss Anspruch 5, worin $R^5$ Alkyl, vorzugsweise Methyl, und $R^6$ den Rest einer gegebenenfalls acylierten Aminosäure oder eines gegebenenfalls acylierten Dipeptids, vorzugsweise den acylierten Rest von Histidin oder Phenylalanin oder den acylierten Rest des Dipeptids aus Histidin und Phenylalanin, oder $R^5$ und $R^6$ zusammen mit dem sie verbindenden Stickstoffatom ein 5- oder 6-gliedriges

Lactam bedeuten.

7. Verfahren gemäss einem der Ansprüche 1-6, worin A die Gruppe (a) bedeutet.

8. Verfahren gemäss einem der Ansprüche 1-7, worin $R^7$ Phenyl oder substituiertes Phenyl, vorzugsweise Phenyl, bedeutet.

9. Verfahren gemäss einem der Ansprüche 1-8, worin $R^8$ Alkylcarbonylalkyl, Aminoalkylcarbonylalkyl, substituiertes Aminoalkylcarbonylalkyl, Aminoalkylsulfonylalkyl, substituiertes Aminoalkylsulfonylalkyl oder Alkylsulfonylalkyl, vorzugsweise Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeutet.

10. Verfahren gemäss einem der Ansprüche 1-6, worin A die Gruppe (b) bedeutet, worin Y den N-terminal mit Z verbundenen. bivalenten Rest von Phenylalanin bedeutet.

11. Verfahren gemäss einem der Ansprüche 1-6 und 10, worin Z die Grupe $R^a$-O-CO- bedeutet, worin $R^a$ einen gegebenenfalls substituierten, gesättigten aliphatischen Kohlenwasserstoffrest mit bis zu 10 Kohlenstoffatomen oder einen gegebenenfalls substituierten heteroaromatischen Kohlenwasserstoffrest mit bis zu 18 Kohlenstoffatomen, vorzugsweise einen gesättigten, aliphatishen Kohlenwasserstoffrest mit bis zu 6 Kohlenstoffatomen oder einen heteroaromatischen Rest mit bis zu 10 Kohlenstoffatomen, bedeutet.

12. Verfahren gemäss einem der Ansprüche 1-9, worin $R^1$ Wasserstoff, $R^2$ Imidazol-4-yl, $R^3$ Cyclohexylmethyl, $R^4$ die Gruppe -N($R^5$)($R^6$), $R^5$ Methyl, $R^6$ den acylierten Rest von Histidin oder Phenylalanin oder den acylierten Rest des Dipeptids aus Histidin und Phenylalanin, $R^7$ Phenyl und $R^8$ $C_1$-$C_4$-Alkylcarbonylmethyl oder $C_1$-$C_4$-Alkylsulfonylmethyl bedeuten.

13. Verfahren gemäss Anspruch 1, dadurch gekennzeichnet, dass man (S)-N-[(1S,2S)-1-(Cyclohexylmethyl)-2-hydroxy-3-(2-oxopiperidino)propyl]-α-[(R)-α-(3,3-dimethyl-2-oxobutyl)-hydrocinnamamido]imidazol-4-propionamid herstellt.

14. Verfahren gemäss Anspruch 1, dadurch gekennzeichnet, dass man (S)-N-[(1S,2S)-1-(Cyclohexylmethyl)-3-[(S)-α-[(R)-α-(3,3-dimethyl-2-oxobutyl)hydrocinnamamido]-N-isopropylimidazol-4-propionamido]-2-hydroxypropyl]-α-[(R)-α-(3,3-dimethyl-2-oxobutyl)hydrocinnamamido]imidazol-4-propionamid herstellt.

15. Verfahren gemäss Anspruch 1, dadurch gekennzeichnet, dass man (S)-N-[(1S,2S)-1-(Cyclohexylmethyl)-2-hydroxy-3-[[(S)-1-[(S)-α-(3,3-dimethyl-2-oxobutyl)hydrocinnamamido]-2-imidazol-4-yläthyl]methylamino]propyl]-α-[(R)-α-(3,3-dimethyl-2-oxobutyl)hydrocinnamamido]imidazol-4-propionamid herstellt.

16. Verfahren gemäss Anspruch 1, dadurch gekennzeichnet, dass man (S)-N-[(1S,2S)-1-(Cyclohexylmethyl)-2-hydroxy-3-[(S)-3-(imidazol-4-yl)-2-hydrocinnamamido-N-methylpropionamido]propyl]-α-[(R)-α-(3,3-dimethyl-2-oxobutyl)hydrocinnamamido]imidazol-4-propionamid herstellt.

17. Verfahren gemäss Anspruch 1, dadurch gekennzeichnet, dass man t-Butyl [(S)-α-[[(S)-1-[[(1S,2S)-3-[[(S)-1-[(S)-α-(1-t-butoxyformamido)hydrocinnamamido]-2-imidazol-4-yläthyl]methylcarbamoyl]-1-(cyclohexylmethyl)-2-hydroxypropyl]carbamoyl]-2-imidazol-4-yl-äthyl]carbamoyl]phenäthyl]carbamat herstellt.

18. Verfahren gemäss Anspruch 1, dadurch gekennzeichnet, dass man t-Butyl [(S)-α-[[(2S,3S)-3-[(S)-2[-(S)-α-(1-t-butoxyformamido)hydrocinnamamido]-3-imidazol-4-ylpropionamido]-4-cyclohexyl-2-hydroxybutyl]-methylcarbamoyl]phenäthyl]carbamat herstellt.

19. Verfahren gemäss Anspruch 1, dadurch gekennzeichnet, dass man (S)-N-[(1S,2S)-1-(Cyclohexylmethyl)-2-hydroxy-3-phthalimidopropyl]-α-[(R)-α-(3,3-dimethyl-2-oxobutyl)-hydrocinnamamido]-imidazol-4-propionamid herstellt.

20. Verfahren zur Herstellung von Arzneimitteln, insbesondere zur Verwendung bei der Bekämpfung bzw. Verhütung von Bluthochdruck und Herzinsuffizienz, dadurch gekennzeichnet, dass man ein Aminosäurederivat der Formel I in Anspruch 1, in Form eines optisch reinen Diastereomeren, eines Diastereomerengemisches, eines diastereomeren Racemates oder eines Gemisches von diastereomeren Racematen, oder ein pharmazeutisch verwendbares Salz einer solchen Verbindung in eine galenische Darreichungsform bringt.

21. Verwendung eines Aminosäurederivates der Formel I in Anspruch 1, in Form eines optisch reinen Diastereomeren, eines Diastereomerengemisches, eines diastereomeren Racemates oder eines Gemisches von diastereomeren Racematen, oder eines pharmazeutisch verwendbaren Salzes einer solchen Verbindung zur Herstellung von Mitteln gegen Bluthochdruck und/oder Herzinsuffizienz.

22. Verbindungen der allgemeinen Formeln

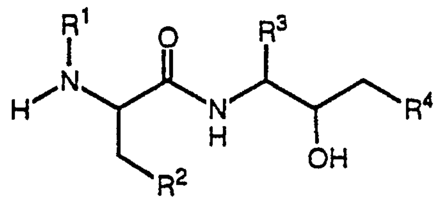

II

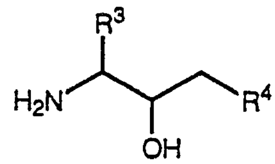

III

und

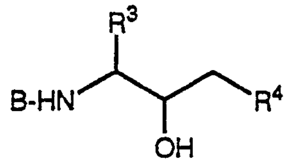

V

worin B eine Aminoschutzgruppe, vorzugsweise t-Butoxycarbonyl oder Benzyloxycarbonyl, $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl, Aethoxycarbonyl, t-Butylcarbonylmethyl, Benzyloxycarbonylmethyl oder t-Butoxy, $R^3$ Isobutyl, Cyclohexylmethyl oder Benzyl, $R^4$ Nitro, Amino oder eine Gruppe der Formel $-N(R^5)(R^6)$ bedeuten, worin $R^5$ Alkyl, Alkoxyalkyl oder gegebenenfalls substituiertes Phenyl, Phenylalkyl oder Phenylsulfonylalkyl und $R^6$ Alkyl, Alkoxyalkyl, gegebenenfalls substituiertes Phenyl, Phenylalkyl oder Phenylsulfonylalkyl, Alkanoyl, Alkoxycarbonyl, Arylalkoxycarbonyl, gegebenenfalls substituiertes Benzimidazolonyl oder den Rest einer gegebenenfalls acylierten Aminosäure oder eines gegebenenfalls acylierten Dipeptids oder $R^5$ und $R^6$ zusammen mit dem sie verbindenden Stickstoffatom einen 5- oder 6-gliedrigen Heterocyclus, ein 5- oder 6-gliedriges Lactam oder ein 5- oder 6-gliedriges Imid bedeuten.